(19) Europäisches Patentamt
European Patent Office
Office européen des brevets

(11) Veröffentlichungsnummer: **0 555 825 A1**

# (12) EUROPÄISCHE PATENTANMELDUNG

(21) Anmeldenummer: **93102053.1**

(22) Anmeldetag: **10.02.93**

(51) Int. Cl.[5]: **C07D 471/04**, A61K 31/435, C07F 9/6561, //(C07D471/04, 235:00,221:00)

(30) Priorität: **11.02.92 DE 4203872**

(43) Veröffentlichungstag der Anmeldung: **18.08.93 Patentblatt 93/33**

(84) Benannte Vertragsstaaten: **AT BE CH DE DK ES FR GB GR IE IT LI LU NL PT SE**

(71) Anmelder: **Dr. Karl Thomae GmbH**

**D-88397 Biberach(DE)**

(72) Erfinder: **Ries, Uwe, Dr. Dipl.-Chem.**
**Dunantstrasse 10**
**W-7950 Biberach 1(DE)**
Erfinder: **Hauel, Norbert, Dr. Dipl.-Chem.**
**Marderweg 12**
**W-7957 Schemmerhofen(DE)**
Erfinder: **Narr, Berthold, Dr. Dipl.-Chem.**
**Obere Au 5**
**W-7950 Biberach 1(DE)**
Erfinder: **van Meel, Jacques, Dr.**
**Schubertweg 4**
**W-7951 Mittelbiberach(DE)**
Erfinder: **Wienen, Wolfgang, Dr. Dipl.-Biol.**
**Am Schiessberg 28**
**W-7951 Äpfingen(DE)**
Erfinder: **Entzeroth, Michael, Dr. Dipl.-Chem.**
**Sebastian-Sailer-Strasse 44**
**W-7951 Warthausen(DE)**

## (54) Imidazo 1,2-a pyridine als Angiotensin-II-Antagonisten.

(57) Die Erfindung betrifft Imidazo[1,2-a]pyridine der allgemeinen Formel

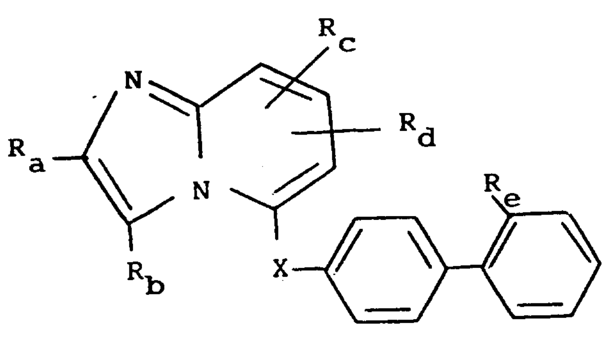

,(I)

in der
$R_a$ bis $R_e$ wie im Anspruch 1 definiert sind, deren Enantiomere und deren Salze, welche wertvolle Eigenschaften aufweisen.

Die neuen Verbindungen stellen insbesondere Angiotensin-Antagonisten dar.

EP 0 555 825 A1

Gegenstand der vorliegenden Erfindung sind neue Imidazo[1,2-a]pyridine der allgemeinen Formel

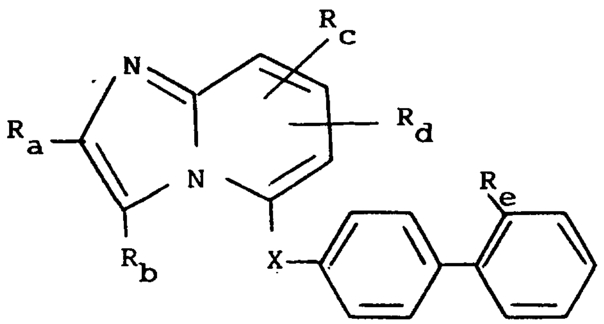

,(I)

deren Enantiomeren und deren Salze, insbesondere für die pharmazeutische Anwendung deren physiologisch verträglichen Salze mit anorganischen oder organischen Säuren oder Basen.

In der obigen allgemeinen Formel I bedeutet

$R_a$ eine geradkettige oder verzweigte Alkylgruppe mit 1 bis 6 Kohlenstoffatomen, eine Cycloalkylgruppe, eine Alkylgruppe, die durch eine Alkoxygruppe substituiert ist, oder eine Alkoxygruppe mit 1 bis 4 Kohlenstoffatomen,

$R_b$ ein Wasserstoff-, Fluor-, Chlor- oder Bromatom, eine Alkyl-, Hydroxymethyl-, Trifluormethyl-, Formyl-, Carboxy-, Alkoxycarbonyl-, Cyano-, Nitro-, $NH_2CH_2$-, $R_1NHCH_2$- oder $R_1NR_2CH_2$-Gruppe, wobei

$R_1$ und $R_2$, die gleich oder verschieden sein können, Alkylgruppen mit 1 bis 6 Kohlenstoffatomen, Cycloalkyl-, Cycloalkylalkyl-, Phenyl- oder Phenylalkylgruppen oder

$R_1$ und $R_2$ zusammen eine n-Alkylengruppe mit 4 bis 6 Kohlenstoffatomen darstellen,

$R_c$ ein Wasserstoff-, Fluor-, Chlor- oder Bromatom, eine gegebenenfalls durch eine Alkoxy- oder Phenylalkoxygruppe substituierte Alkylgruppe, eine Alkoxy-, Phenylalkoxy-, Trifluormethyl-, $H_2N$-, $R_1NH$- oder $R_1NR_2$-Gruppe, wobei $R_1$ und $R_2$ wie vorstehend erwähnt definiert sind,

$R_d$ ein Wasserstoffatom oder eine Alkylgruppe,

$R_e$ eine Carboxygruppe, eine in-vivo in eine Carboxygruppe überführbare Gruppe, eine Cyano-, 1H-Tetrazolyl-, 1-Triphenylmethyl-tetrazolyl-, Alkansulfonylaminocarbonyl-, Phenylsulfonylaminocarbonyl-, Phenylalkansulfonylaminocarbonyl-, Trifluormethansulfonylaminocarbonyl-, Phosphino-, O-Alkyl-phosphino-, O-Aralkyl-phosphino-, Phosphono-, O-Alkyl-phosphono-, O-Aralkyl-phosphono-, O,O-Dialkylphosphono-, Phosphono-methyl-, O-Alkyl-phosphono-methyl-, O-Aralkyl-phosphono-methyl-, O-Aryl-phosphono-methyl-, O,O-Dialkyl-phosphono-methyl-, Phosphato-, O-Alkyl-phosphato-, O-Aralkyl-phosphato-, O-Aryl-phosphato- oder O,O-Dialkyl-phosphorylgruppe,

X ein Sauerstoffatom, eine gegebenenfalls durch eine Formyl-, $R_1$- oder $R_1CO$-Gruppe substituierte Iminogruppe, eine -CO-, -(HON=C)- oder -($R_3CR_4$)- Gruppe, wobei

$R_3$ ein Wasserstoffatom oder eine Alkylgruppe und

$R_4$ ein Wasserstoffatom, eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe, wobei die Heteroarylgruppe über eine Kohlenstoff-Kohlenstoff-Bindung mit der Alkoxygruppe verknüpft ist,

eine in 2-, 3- oder 4-Stellung durch eine Heteroarylgruppe substituierte Alkoxygruppe, wobei die Heteroarylgruppe über eine Kohlenstoff-Stickstoff-Bindung mit der Alkoxygruppe verknüpft ist,

eine Hydroxy-, Dialkylphosphonomethoxy-, Azido-, CHO-O-, $R_1O$-, $R_5NR_6$-, $R_1CO$-O-, $R_1O$-CO-O-, CHO-$NR_5$-, $R_1$-CO-$NR_7$-, $R_1O$-CO-$NR_5$-, $R_5NR_6$-CO-O-, $R_1SO_2$-O-, $R_5NR_6$-CO-$NR_5$- oder $R_1SO_2$-$NR_7$-Gruppe oder

$R_3$ und $R_4$ zusammen eine 1,2-Ethylendioxy- oder 1,3-n-Propylendioxygruppe darstellen, wobei in den vorstehend erwähnten Gruppen $R_1$ wie vorstehend erwähnt definiert ist, $R_5$ und $R_6$, die gleich oder verschieden sein können, Wasserstoffatome oder die für $R_1$ und $R_2$ eingangs erwähnten Bedeutungen besitzen,

$R_7$ ein Wasserstoffatom oder eine Alkylgruppe oder $R_1$ und $R_7$ zusammen eine n-Alkylengruppe mit 3 bis 5 Kohlenstoffatomen darstellen,

wobei, soweit nichts anderes erwähnt wurde, ein vorstehend erwähnter Alkyl- oder Alkoxyteil jeweils 1 bis 4 Kohlenstoffatome sowie ein vorstehend erwähnter Cycloalkylteil jeweils 3 bis 7 Kohlenstoffatome enthalten kann, und

unter "eine Arylgruppe" eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkanoylamino-, Cyano-,

Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituierte Phenylgruppe, wobei der Alkylteil jeweils 1 bis 4 Kohlenstoffatome enthalten kann, oder eine Naphthylgruppe und unter einer "Heteroarylgruppe" ein über ein Kohlenstoffatom oder über eine Iminogruppe gebundener 5-gliedriger heteroaromatischer Ring, der eine Iminogruppe, ein Sauerstoff- oder Schwefelatom oder eine Iminogruppe und ein Sauerstoff-, Schwefel- oder Stickstoffatom enthält, oder ein über ein Kohlenstoffatom gebundener 6-gliedriger heteroaromatischer Ring, der 1 oder 2 Stickstoffatome enthält, wobei die vorstehend erwähnten heteroaromatischen Ringe im Kohlenstoffgerüst durch eine Alkylgruppe mit 1 bis 6 Kohlenstoffatomen oder durch eine Phenylalkylgruppe substituiert sein können und sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom, durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Fluormethyl-, Difluormethyl-, Trifluormethyl-, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können sowie zwei Methylsubstituenten in 1,2-Stellung zueinander durch eine Methylen- oder Ethylenbrücke miteinander verknüpft sein können und eine gegebenenfalls in einem Imidazolring vorhandene NH-Gruppe durch eine Alkylgruppe mit 1 bis 6 Kohlenstoffatomen, durch eine Phenylalkylgruppe oder durch eine Cycloalkylgruppe substituiert sein kann, zu verstehen ist.

Die neuen Verbindungen der obigen allgemeinen Formel I, in denen $R_e$ eine tert.-Butoxycarbonyl, Cyano- oder 1-Triphenylmethyl-tetrazolylgruppe darstellt, stellen wertvolle Zwischenprodukte dar und die übrigen Verbindungen der obigen allgemeinen Formel I sowie deren physiologisch verträglichen Salze weisen wertvolle pharmakologische Eigenschaften auf, da diese Angiotensin-Antagonisten, insbesondere AngiotensinII-Antagonisten, darstellen.

Gegenstand der vorliegenden Erfindung sind somit die neuen vorstehend erwähnten Imidazo[1,2-a]-pyridine sowie

neue Arzneimittel, welche eine der oben erwähnten pharmakologisch wirksamen Verbindungen der allgemeinen Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition der Reste $R_a$ bis $R_e$ und $R_1$ bis $R_7$ eingangs erwähnten Bedeutungen kommt beispielsweise für

$R_a$ die Bedeutung der Methyl-, Ethyl-, n-Propyl-, Isopropyl-, n-Butyl-, Isobutyl-, tert.-Butyl-, n-Pentyl-, Isopentyl-, Neopentyl-, n-Hexyl-, Isohexyl-, 1,1-Dimethyl-butyl-, 2,2-Dimethyl-butyl-, 3,3-Dimethyl-butyl-, Cyclopropyl-, Cyclobutyl-, Cyclopentyl-, Cyclohexyl-, Cycloheptyl-, Methoxy-methyl-, 2-Methoxy-ethyl-, 3-Methoxy-n-propyl-, Ethoxy-methyl-, 2-Ethoxy-ethyl-, 3-Ethoxy-n-propyl-, n-Propyloxy-methyl-, 2-(n-Propyloxy)ethyl-, 3-(n-Propyloxy)-n-propyl-, Isopropyloxy-methyl, 2-Isopropyloxy-ethyl-, 3-Isopropyloxy-n-propyl-, Methoxy-, Ethoxy-, n-Propyloxy-, Isopropyloxy-, n-Butyloxy-, Isobutyloxy- oder tert.Butyloxygruppe,

für $R_b$ die des Wasserstoff-, Fluor-, Chlor- oder Bromatoms, der Cyano-, Nitro-, Formyl-, Carboxy-, Methoxycarbonyl-, Ethoxycarbonyl-, n-Propoxycarbonyl-, Isopropoxycarbonyl-, n-Butoxycarbonyl-, Isobutoxycarbonyl-, tert.Butoxycarbonyl-, Trifluormethyl-, Methyl-, Ethyl-, n-Propyl-, Isopropyl-, Hydroxymethyl-, Aminomethyl-, Dimethylaminomethyl-, Diethylaminomethyl-, Di-n-propylaminomethyl-, Diisopropylaminomethyl-, Di-n-butylaminomethyl-, Di-isobutylaminomethyl-, (Pyrrolidin-1-yl)-methyl-, (Piperidin-1-yl)-methyl- oder Morpholinomethylgruppe,

für $R_c$ die des Wasserstoff-, Fluor-, Chlor- oder Bromatoms, der Methoxy-, Ethoxy-, n-Propyloxy-, Isopropyloxy-, Benzyloxy-, 2-Phenylethyloxy-, 3-Phenyl-n-propyloxy-, Methyl-, Ethyl-, n-Propyl-, Isopropyl-, Methoxymethyl-, 2-Methoxyethyl-, Ethoxymethyl-, 2-Ethoxyethyl-, n-Propyloxymethyl-, 2-(n-Propyloxy)ethyl-, Isopropyloxymethyl-, Benzyloxymethyl-, 2-Benzyloxyethyl-, 2-Phenylethyloxymethyl-, Trifluormethyl-, Dimethylamino-, Diethylamino-, Di-n-Propylamino-, Diisopropylamino-, Pyrrolidin-1-yl- oder Piperidin-1-yl-gruppe,

für $R_d$ die des Wasserstoffatoms, der Methyl-, Ethyl-, n-Propyl-, Isopropyl-, n-Butyl-, Isobutyl- oder tert.Butylgruppe,
für $R_e$ die der Carboxy-, Methoxycarbonyl-, Ethoxycarbonyl-, n-Propyloxycarbonyl-, Isopropyloxycarbonyl-, n-Butyloxycarbonyl-, Isobutyloxycarbonyl-, tert.Butyloxycarbonyl-, n-Pentyloxycarbonyl-, n-Hexyloxycarbonyl-, Benzyloxycarbonyl-, 1-Phenylethoxycarbonyl-, 2-Phenylethoxycarbonyl-, 3-Phenylpropyloxycarbonyl-, Acetoxymethyloxycarbonyl-, Propionyloxymethoxycarbonyl-, n-Butyryloxymethoxycarbonyl-, Isobutyryloxymethoxycarbonyl-, 1-(Acetyloxy)ethoxycarbonyl-, 1-(Isobutyryloxy)ethoxycarbonyl-, Benzoyloxymethoxycarbonyl-, Cinnamyloxycarbonyl-, Pivaloyloxymethoxycarbonyl-, 1-(Ethoxycarbonyloxy)-ethoxycarbonyl-, Methoxymethoxycarbonyl-, Cyclohexyloxycarbonylmethoxycarbonyl-, 1-(Cyclohexyloxycarbonyloxy)ethoxycarbonyl-, Cyclopentylcarbonyloxymethoxycarbonyl-, (1,3-Dioxa-2-oxo-4-methyl-cyclopenten-5-yl)methoxycarbonyl-, Cyano-, 1H-Tetrazolyl-, 1-Triphenylmethyl-tetrazolyl-, Methylsulfonylaminocarbonyl-, Ethylsulfonylaminocarbonyl-, n-Propylsulfonylaminocarbonyl-, Isopropylsulfonylaminocarbonyl-, Phenylsulfonylaminocarbonyl-, Benzylsulfonylaminocarbonyl-, 1-Phenylethylsulfonylaminocarbonyl-, 2-Phenyl-ethylsulfonylaminocarbonyl-,1-Phenyl-n-propylsulfonylaminocarbonyl-, 2-Phenyl-n-propylsulfonylaminocarbonyl-, 3-Phenyl-n-propylsulfonylaminocarbonyl-, Trifluormethansulfonylaminocarbonyl-,Phosphino-, O-Methyl-phosphino-, O-Ethyl-phosphino-, O-n-Propyl-phosphino-, O-Isopropyl-phosphino-,O-Benzyl-phosphino-, Phosphono-, O-Methyl-phosphono-, O-Ethyl-phosphono-, O-n-Propyl-phosphono-, O-Isopropyl-phosphono-,O-Benzyl-phosphono-, O,O-Dimethyl-phosphono, O,O-Diethyl-phosphono-, O,O-Di-(n-propyl)phosphono-, O,O-Di-(isopropyl)phosphono-, Phosphonomethyl-, O-Methyl-phosphonomethyl-, O-Ethyl-phosphonomethyl-, O-n-Propyl-phosphonomethyl-, O-Isopropyl-phosphonomethyl-, O-Benzyl-phosphonomethyl-, O,O-Dimethyl-phosphonomethyl-, O,O-Diethyl-phosphonomethyl-, O,O-Di-(n-propyl)phosphonomethyl-, O,O-Di-(isopropyl)phosphonomethyl-,Phosphato-, O-Methyl-phosphato-, O-Ethyl-phosphato-, O-n-Propyl-phosphato-, O-Isopropyl-phosphato-, O-Benzyl-phosphato-, O-Phenyl-phosphato-, O,O-Dimethyl-phosphoryl-, O,O-Diethyl-phosphoryl-, O,O-Di-(n-propyl)-phosphoryl- oder O,O-Di-(isopropyl)phosphorylgruppe,
für $R_1$ und $R_2$ jeweils die der Methyl-, Ethyl-, n-Propyl-, Isopropyl-, n-Butyl-, Isobutyl-, tert.Butyl-, n-Pentyl-, Isopentyl-, Neopentyl-, n-Hexyl-, Isohexyl-, Cyclopropyl-, Cyclobutyl-, Cyclopentyl-, Cyclohexyl-, Cycloheptyl-, Cyclopropylmethyl-, 2-Cyclopropylethyl-, 3-Cyclopropyl-n-propyl-, 4-Cyclopropyl-n-butyl-, Cyclobutylmethyl-, 2-Cyclobutylethyl-, 3-Cyclobutyl-n-propyl-, 4-Cyclobutyl-n-butyl-, Cyclopentylmethyl-, 2-Cyclopentylethyl-, 3-Cyclopentyl-n-propyl-, 4-Cyclopentyl-n-butyl-, Cyclohexylmethyl-, 2-Cyclohexylethyl-, 3-Cyclohexyl-n-propyl-, 4-Cyclohexyl-n-butyl-, Cycloheptylmethyl-, 2-Cycloheptylethyl-, 3-Cycloheptyl-n-propyl-, 4-Cycloheptyl-n-butyl-, Phenyl-, Phenylmethyl-, 1-Phenylethyl-, 2-Phenylethyl-, 1-Phenyl-n-propyl-, 2-Phenyl-n-propyl- oder 3-Phenyl-n-propylgruppe,
für $R_1$ und $R_2$ zusammen die der n-Butylen-, n-Pentylen- oder n-Hexylengruppe,
für $R_3$ die des Wasserstoffatoms, der Methyl-, Ethyl-, n-Propyl-, Isopropyl-, n-Butyl-, Isobutyl- oder tert.-Butylgruppe,
für $R_4$ die des Wasserstoffatoms, Carboxymethoxy-, 2-Carboxy-ethoxy-, 3-Carboxy-propoxy-, 4-Carboxy-butoxy-, Methoxycarbonylmethoxy-, 2-Methoxycarbonyl-ethoxy-, 3-Methoxycarbonyl-propoxy-, 4-Methoxycarbonyl-butoxy-, Ethoxycarbonylmethoxy-, 2-Ethoxycarbonyl-ethoxy-, 3-Ethoxycarbonyl-propoxy-, 4-Ethoxycarbonyl-butoxy-, Isopropyloxycarbonylmethoxy-, 2-n-Butoxycarbonyl-ethoxy-, 3-Isobutoxycarbonyl-propoxy-, 4-tert.Butoxycarbonyl-butoxy-, Aminocarbonylmethoxy-, 2-Aminocarbonyl-ethoxy-, 3-Aminocarbonyl-propoxy-, 4-Aminocarbonyl-butoxy-, Methylaminocarbonylmethoxy-, 2-Methylaminocarbonyl-ethoxy-, 3-Methylaminocarbonyl-propoxy-, 4-Methylaminocarbonyl-butoxy-, Ethylaminocarbonylmethoxy-, 2-Ethylaminocarbonyl-ethoxy-, 3-Ethylaminocarbonyl-propoxy-, 4-Ethylaminocarbonyl-butoxy-, n-Propylaminocarbonylmethoxy-, 2-n-Propylaminocarbonyl-ethoxy-, 3-n-Propylaminocarbonyl-propoxy-, 4-n-Propylaminocarbonyl-butoxy-, Isopropylaminocarbonylmethoxy-, 2-n-Butylaminocarbonyl-ethoxy-, 3-Isobutylaminocarbonyl-propoxy-, 4-Isopropylaminocarbonyl-butoxy, Dimethylaminocarbonylmethoxy-, 2-Dimethylaminocarbonyl-ethoxy-, 3-Dimethylaminocarbonyl-propoxy-, 4-Dimethylaminocarbonyl-butoxy-, Diethylaminocarbonylmethoxy-, 2-Diethylaminocarbonyl-ethoxy-, 3-Diethylaminocarbonyl-propoxy-, 4-Diethylaminocarbonyl-butoxy-, Di-n-propylaminocarbonylmethoxy-, 2-Di-n-propylaminocarbonyl-ethoxy-,3-Di-n-propylaminocarbonyl-propoxy-, O,O-Dimethylphosphono-methoxy, O,O-Diethylphosphono-methoxy-, 4-Di-n-propylaminocarbonyl-butoxy-, Diisopropylaminocarbonylmethoxy-, 2-Di-n-butylaminocarbonyl-ethoxy-, 3-Diisobutylaminocarbonyl-propoxy-, 4-Di-n-butylaminocarbonyl-butoxy-,Azido-, 2-(Imidazol-1-yl)-ethoxy-, 3-(Imidazol-1-yl)-propoxy-, 4-(Imidazol-1-yl)-butoxy-, 2-(Benzimidazol-1-yl)-ethoxy-, 3-(Benzimidazol-1-yl)-propoxy-, 4-(Benzimidazol-1-yl)-butoxy-, Chinolin-2-yl-methoxy-, 2-(Chinolin-2-yl)-ethoxy-, 3-(Chinolin-2-yl)-propoxy-, 4-(Chinolin-2-yl)-butoxy-, Isochinolin-1-yl-methoxy-, 2-(Isochinolin-1-yl)-ethoxy-, 3-(Isochinolin-1-yl)-propoxy-, 4-(Isochinolin-1-yl)-butoxy-, Isochinolin-3-yl-methoxy-, 2-(Isochinolin-3-yl)-ethoxy-, 3-(Isochinolin-3-yl)-propoxy-, 4-(Isochinolin-3-yl)-butoxy-, Pyridin-2-yl-methoxy-,

2-(Pyridin-2-yl)-ethoxy-, 3-(Pyridin-2-yl)-propoxy-, 4-(Pyridin-2-yl)-butoxy-, Pyridin-3-yl-methoxy-, 2-(Pyridin-3-yl)-ethoxy-, 3-(Pyridin-3-yl)-propoxy-, 4-(Pyridin-3-yl)-butoxy-, Pyridin-4-yl-methoxy-, 2-(Pyridin-4-yl)-ethoxy-, 3-(Pyridin-4-yl)-propoxy-, 4-(Pyridin-4-yl)-butoxy-, 4-Methylimidazol-2-yl-methoxy-, 2-(1-Methylimidazol-4-yl)-ethoxy-,2-(1-Methylimidazol-5-yl)-ethoxy-, 2-(1-n-Hexylimidazol-4-yl)-ethoxy-, 2-(1-n-Hexylimidazol-5-yl)-ethoxy-, 1-Benzylimidazol-4-yl-methoxy-, 2-(1-Benzylimidazol-5-yl)-ethoxy-,2-(1,2-Dimethyl-imidazol-4-yl)-ethoxy-, 2-(1,2-Dimethylimidazol-5-yl)-ethoxy-, 2-(2-Methylimidazol-1-yl)-ethoxy-, 2-(2-n-Butyl-4-methyl-imidazol-1-yl)-ethoxy-, 2-(2-n-Butyl-5-methyl-imidazol-1-yl)-ethoxy-, 2-(1-Benzyl-2-methyl-imidazol-4-yl)-ethoxy-, 2-(1-Benzyl-2-methyl-imidazol-5-yl)-ethoxy-, 2-(4,5-Trimethylen-imidazol-2-yl)-ethoxy-, 2-(4,5-Trimethylen-imidazol-1-yl)-ethoxy-, 2-(4,5-Trimethylen-2-methyl-imidazol-1-yl)-ethoxy-, 2-(Benzimidazol-2-yl)-ethoxy-, 2-(1-Methylbenzimidazol-2-yl)-ethoxy-, 2-(1-Ethylbenzimidazol-2-yl)-ethoxy-, 2-(1-n-Propylbenzimidazol-2-yl)-ethoxy-, 2-(1-Isopropylbenzimidazol-2-yl)-ethoxy-, 2-(1-n-Butylbenzimidazol-2-yl)-ethoxy-, 2-(1-Isobutylbenzimidazol-2-yl)-ethoxy-, 2-(1-n-Pentylbenzimidazol-2-yl)-ethoxy-, 2-(1-n-Hexylbenzimidazol-2-yl)-ethoxy-, 2-(5-Nitro-benzimidazol-1-yl)-ethoxy-, 2-(5-Amino-benzimidazol-2-yl)-ethoxy-, 2-(5-Acetamido-benzimidazol-2-yl)-ethoxy-, 2-(5-Methyl-benzimidazol-2-yl)-ethoxy-, 2-(5-Methoxy-benzimidazol-2-yl)-ethoxy-, 2-(5-Ethoxy-benzimidazol-2-yl)-ethoxy-, 2-(5-Methyl-benzimidazol-1-yl)-ethoxy-, 2-(6-Methyl-benzimidazol-1-yl)-ethoxy-, 2-(4-Methyl-benzimidazol-1-yl)-ethoxy-, 2-(5-Chlor-benzimidazol-2-yl)-ethoxy-, 2-(5-Chlor-benzimidazol-1-yl)-ethoxy-, 2-(6-Chlor-1-methyl-benzimidazol-2-yl)-ethoxy-, 2-(5,6-Dimethoxy-1-methyl-benzimidazol-2-yl)-ethoxy-, 2-(5,6-Dimethoxy-benzimidazol-1-yl)-ethoxy-, 2-(5--Fluor-1-methyl-benzimidazol-2-yl)-ethoxy-, 2-(6-Fluor-benzimidazol-1-yl)-ethoxy-, 2-(5-Trifluormethyl-benzimidazol-2-yl)-ethoxy-, 2-(5-Trifluormethyl-benzimidazol-1-yl)-ethoxy-, 2-(5-Carboxyl-1-methyl-benzimidazol-2-yl)-ethoxy-, 2-(5-Aminocarbonyl-benzimidazol-2-yl)-ethoxy-, 2-(5-Aminocarbonyl-benzimidazol-1-yl)-ethoxy-, 2-(5-Methoxycarbonyl-1-methyl-benzimidazol-2-yl)-ethoxy-, 2-(5-Methylaminocarbonyl-1-methylbenzimidazol-2-yl)-ethoxy-, 2-(5-Methylaminocarbonyl-benzimidazol-1-yl)-ethoxy-, 2-(4,5,6,7-Tetrahydro-benzimidazol-1-yl)-ethoxy-, 2-(4,5,6,7-Tetrahydro-1-methyl-benzimidazol-2-yl)-ethoxy-, 2-(4,5,6,7-Tetrahydro-1-ethyl-benzimidazol-2-yl)-ethoxy-, 2-(4,5,6,7-Tetrahydro-1-n-butyl-benzimidazol-2-yl)-ethoxy-, 2-(4,5,6,7-Tetrahydro-1-n-hexyl-benzimidazol-2-yl)-ethoxy-, (Imidazo[1,2-a]pyridin-2-yl)-methoxy-, (5-Methyl-imidazo[1,2-a]pyridin-2-yl)-methoxy-, (6-Methyl-imidazo-[1,2-a]pyridin-2-yl)-methoxy-, (7-Methyl-imidazo[1,2-a]pyridin-2-yl)-methoxy-, (8-Methyl-imidazo[1,2-a]pyridin-2-yl)-methoxy-, (5,7-Dimethyl-imidazo[1,2-a]pyridin-2-yl)-methoxy-, (6-Aminocarbonyl-imidazo[1,2-a]pyridin-2-yl)-methoxy-, (6-Chlor-imidazo[1,2-a]pyridin-2-yl)-methoxy-, (6-Brom-imidazo[1,2-a]pyridin-2-yl)-methoxy-, (5,6,7,8-Tetrahydro-imidazo[1,2-a]pyrimidin-2-yl)-methoxy-, (Imidazo[1,2-a]pyrimidin-2-yl)-methoxy-, (5,7-Dimethyl-imidazo[1,2-a]pyridin-2-yl)-methoxy-, 2-(Imidazo[4,5-b]pyridin-2-yl)-ethoxy-, 2-(1-Methyl-imidazo[4,5-b]pyridin-2-yl)-ethoxy-, 2-(Imidazo-[4,5-b]pyridin-1-yl)-ethoxy-, 2-(4-Methyl-imidazo[4,5-b]pyridin-2-yl)-ethoxy-, 2-(6-Methyl-imidazo[4,5-b]pyridin-2-yl)-ethoxy-, 2-(4-Methyl-imidazo[4,5-b]pyridin-1-yl)-ethoxy-, 2-(6-Methyl-imidazo[4,5-b]pyridin-1-yl)-ethoxy-, 2-(Imidazo-[4,5-c]pyridin-1-yl)-ethoxy, 2-(1-Methyl-imidazo[4,5-c]pyridin-2-yl)-ethoxy-, 2-(1-n-Hexyl-imidazo[4,5-c]-pyridin-2-yl)-ethoxy-, 2-(Imidazo[2,1-b]thiazol-6-yl)-ethoxy-, 2-(3-Methyl-imidazo[2,1-b]thiazol-6-yl)-ethoxy-, 2-(2-Phenyl-imidazo-[2,1-b]thiazol-6-yl)-ethoxy-, 2-(3-Phenyl-imidazo[2,1-b]thiazol-6-yl)-ethoxy-, 2-(2,3-Dimethyl-imidazo[2,1-b]thiazol-6-yl)-ethoxy-, 2-(Imidazo[1,2-c]pyrimidin-2-yl)-ethoxy-, 2-(Imidazo[1,2-a]-pyrazin-2-yl)-ethoxy-, 2-(Imidazo[1,2-b]-pyridazin-2-yl)-ethoxy-, 2-(Imidazo[4,5-c]pyridin-2-yl)-ethoxy-, 2-(Purin-8-yl)-ethoxy- oder 2-(Purin-9-yl)-ethoxy-gruppe,

für $R_5$ die des Wasserstoffatoms und die für $R_1$ vorstehenderwähnten Bedeutungen,

für $R_6$ die des Wasserstoffatoms und die für $R_1$ vorstehenderwähnten Bedeutungen,

für $R_7$ die des Wasserstoffatoms, der Methyl-, Ethyl-, n-Propyl-, Isopropyl-, n-Butyl- oder Isobutylgruppe,

für die $R_1$-CO-$NR_7$-Gruppe die der Pyrrolidin-2-on-, Piperidin-2-on- oder Hexamethylenimin-2-on-gruppe und

für die $R_1$-$SO_2$-$NR_7$-Gruppe die der Propansultam-, Butansultam- oder Pentansultamgruppe in Betracht.

Bevorzugte Verbindungen der allgemeinen Formel I sind diejenigen, in denen

$R_a$ eine geradkettige oder verzweigte Alkylgruppe mit 1 bis 4 Kohlenstoffatomen, eine Cyclopropyl-, Cyclobutyl-, Alkoxy-, Methoxymethyl- oder Ethoxymethylgruppe,

$R_b$ ein Wasserstoff-, Chlor- oder Bromatom, eine Alkyl-, Aminomethyl-, $R_1NHCH_2$- oder $R_1NR_2CH_2$-Gruppe, wobei

$R_1$ und $R_2$, die gleich oder verschieden sein können, Alkylgruppen mit 1 bis 4 Kohlenstoffatomen, Cyclohexyl-, Phenyl- oder Benzylgruppen oder

$R_1$ und $R_2$ zusammen eine n-Butylengruppe darstellen,

$R_c$ ein Wasserstoff-, Chlor- oder Bromatom, eine Alkyl- oder Trifluormethylgruppe,

$R_d$ ein Wasserstoffatom oder eine Alkylgruppe,

$R_e$ ein Carboxy- oder eine 1H-Tetrazol-5-yl-gruppe,

X ein Sauerstoffatom, eine gegebenenfalls durch eine Formyl-, $R_1$- oder $R_1CO$-Gruppe substituierte

Iminogruppe, eine -CO-, -(HON = C)- oder -($R_3CR_4$)- Gruppe, wobei
$R_3$ ein Wasserstoffatom oder eine Alkylgruppe und
$R_4$ ein Wasserstoffatom, eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe, wobei die Heteroarylgruppe über eine Kohlenstoff-Kohlenstoff-Bindung mit der Alkoxygruppe verknüpft ist,
eine in 2-, 3- oder 4-Stellung durch eine Heteroarylgruppe substituierte Alkoxygruppe, wobei die Heteroarylgruppe über eine Kohlenstoff-Stickstoff-Bindung mit der Alkoxygruppe verknüpft ist,
eine Hydroxy-, $R_1O$-, $R_1CO$-O-, $R_1O$-CO-O-, Azido-, $R_5NR_6$-, CHO-$NR_5$-, $R_1$-CO-$NR_7$-, $R_1O$-CO-$NR_5$-, $R_5NR_6$-CO-O-, $R_1SO_2$-O-, $R_5NR_6$-CO-$NR_5$- oder $R_1SO_2$-$NR_7$-Gruppe darstellen, wobei in den vorstehend erwähnten Gruppen $R_1$ wie vorstehend erwähnt definiert ist,
$R_5$ und $R_6$, die gleich oder verschieden sein können, Wasserstoffatom oder die für $R_1$ eingangs erwähnten Bedeutungen besitzen,
$R_7$ ein Wasserstoffatom oder eine Alkylgruppe oder $R_1$ und $R_7$ zusammen eine n-Alkylengruppe mit 3 bis 5 Kohlenstoffatomen darstellen, bedeuten,
wobei, soweit nichts anderes erwähnt wurde, ein vorstehend erwähnter Alkyl- oder Alkoxyteil jeweils 1 bis 3 Kohlenstoffatome sowie ein vorstehend erwähnter Cycloalkylteil jeweils 3 bis 7 Kohlenstoffatome enthalten kann,
deren Enantiomeren und deren Salze, insbesondere für die pharmazeutische Anwendung deren physiologisch verträglichen Salze mit anorganischen oder organischen Säuren oder Basen,
Besonders bevorzugte Verbindungen der obigen allgemeinen Formel I sind diejenigen, in denen
$R_a$ eine Alkylgruppe mit 2 bis 4 Kohlenstoffatomen,
$R_b$ ein Wasserstoffatom,
$R_c$ ein Wasserstoffatom oder eine Methylgruppe,
$R_d$ ein Wasserstoffatom,
$R_e$ eine Carboxy- oder 1H-Tetrazolylgruppe und
X eine Carbonylgruppe oder eine gegebenenfalls durch eine Hydroxy-, Methoxy-, Benzyloxy-, Pyridylmethoxy-, Acetoxy-, Ethoxycarbonylmethoxy-, Cyclohexylcarbonyloxy- oder Cyclohexylaminocarbonyloxygruppe substituierte Methylengruppe bedeuten,
deren Enantiomeren und deren Salze, insbesondere für die pharmazeutische Anwendung deren physiologisch verträglichen Salze mit anorganischen oder organischen Säuren oder Basen,
Erfindungsgemäß erhält man die Verbindungen nach folgenden Verfahren:

a) Zur Herstellung einer Verbindung der allgemeinen Formel I, in der X eine -(HO-$CR_3$)- Gruppe und $R_b$ ein Wasserstoff-, Fluor- oder Chloratom, eine Alkyl- oder Trifluormethylgruppe darstellt:
Umsetzung eines Imidazo[1,2-a]pyridins der allgemeinen Formel

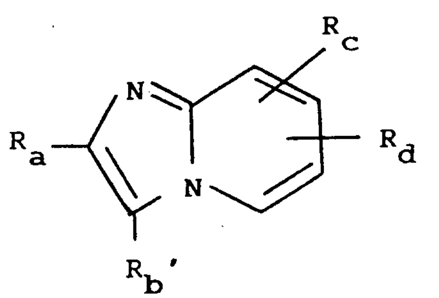

,(II)

in der
$R_a$, $R_c$ und $R_d$ wie eingangs definiert sind und
$R_b$' ein Wasserstoff-, Fluor- oder Chloratom, eine Alkyl- oder Trifluormethylgruppe darstellt, mit einer Biphenylverbindung der allgemeinen Formel

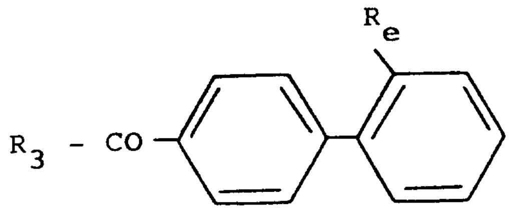
,(III)

in der
$R_e$ und $R_3$ wie eingangs definiert sind.

Die Umsetzung erfolgt unter Einwirkung einer starken Base wie Kalium-tert.butylat, Natriumhydrid, Lithiumamid, Natriumamid, Lithiumdiisopropylamid, Lithium-2,2,6,6-tetramethylpiperidid, Lithiumhexamethyldisilazid oder einer Lithiumorganischen Verbindung wie Methyllithium, Ethyllithium, n-Butyllithium, sec.-Butyllithium-, tert.-Butyllithium, Phenyllithium oder gegebenenfalls einer äquimolaren Mischung von n-Butyllithium mit Kalium-tert.-butylat, n-Butyllithium mit N,N,N',N'-Tetramethylenethylendiamin oder n-Butyllithium mit N,N,N',N'-Tetramethylenethylendiamin und Kalium-tert.butylat (siehe beispielsweise "Lambert Brandsma, Hermann Verkruisse, Preparative Polar Organometallic Chemistry 1, Springer Verlag 1987") auf eine Verbindung der allgemeinen Formel II, zweckmäßigerweise in einem Lösungsmittel oder Lösungsmittelgemisch wie Diethylether, Tetrahydrofuran, Dioxan oder flüssigem Ammoniak bei Temperaturen zwischen -100 und 20 ° C, vorzugsweise bei Temperaturen zwischen -78 ° C und -20 ° C, und anschließender Umsetzung mit einer Verbindung der allgemeinen Formel III bei -100 ° C bis -50 ° C, vorzugsweise bei -78 ° C, und anschließendem Erwärmen auf 20 ° C bis 50 ° C, vorzugsweise auf Raumtemperatur.

b) Zur Herstellung einer Verbindung der allgemeinen Formel I, in der $R_e$ eine Carboxygruppe darstellt:
Überführung einer Verbindung der allgemeinen Formel

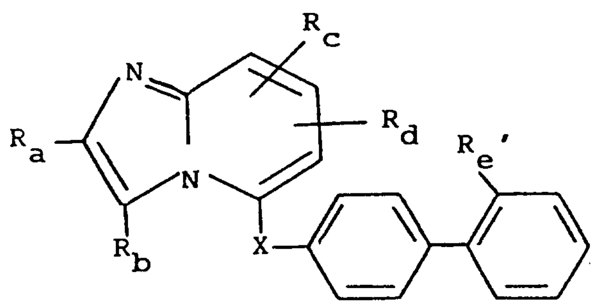
,(IV)

in der
$R_a$ bis $R_d$ und X wie eingangs definiert sind und
$R_e$' eine mittels Hydrolyse, Thermolyse oder Hydrogenolyse in eine Carboxygruppe überführbare Gruppe darstellt, in eine entsprechende Carboxylverbindung.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe, Ester mit tertiären Alkoholen, z.B. der tert. Butylester, mittels Thermolyse in eine Carboxygruppe und Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 ° C und 120 ° C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_e$' in einer Verbindung der allgemeinen Formel IV eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei

Temperaturen zwischen O und 50 °C in die Carboxygruppe übergeführt werden.

Bedeutet $R_e$' in einer Verbindung der allgemeinen Formel IV beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 °C und 100 °C, abgespalten werden.

Bedeutet $R_e$' in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50 °C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenylpropionsäuregruppe, mitreduziert oder durch Wasserstoffatome, z.B. ein Halogenatom durch ein Wasserstoffatom, ersetzt werden.

c) Zur Herstellung einer Verbindung der allgemeinen Formel I, in der X eine Carbonylgruppe darstellt: Oxidation einer Verbindung der allgemeinen Formel

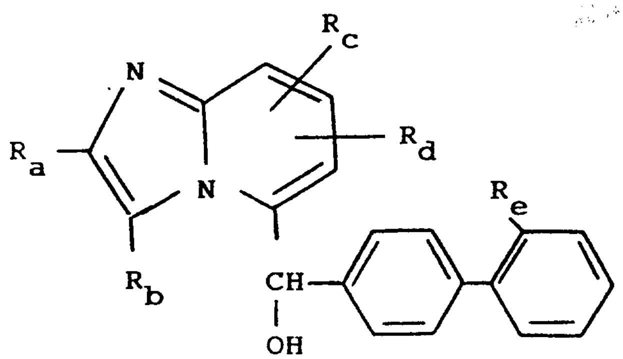

,(V)

in der

$R_a$ bis $R_e$ wie eingangs definiert sind.

Die Oxidation wird vorzugsweise in einem Lösungsmittel oder Lösungsmittelgemisch, z. B. in Aceton, Pyridin, Wasser/Pyridin, Eisessig, Dichlormethan oder Chloroform bei Temperaturen zwischen -20 und 100 °C durchgeführt. Als Oxidationsmittel kommen z. B. Chromsäure in Eisessig oder in Aceton oder modifizierte Chromsäure-Reagentien wie Pyridiniumchlorochromat oder Pyridiniumdichromat in Dichlormethan, Mangandioxid in Chloroform, Kaliumpermanganat in Eisessig, Pyridin oder in Aceton, Natriumhypochlorit in Eisessig, Dimethylsulfoxid in Kombination mit Acetanhydrid, Trifluoracetanhydrid, Dicyclohexylcarbodiimid oder in Kombination mit Oxalylchlorid und Triethylamin, vorzugsweise jeweils bei Temperaturen zwischen 0 und 20 °C in Betracht.

d) Zur Herstellung einer Verbindung der allgemeinen Formel I, in der X eine -($R_3$CH)- Gruppe darstellt: Reduktion einer Verbindung der allgemeinen Formel

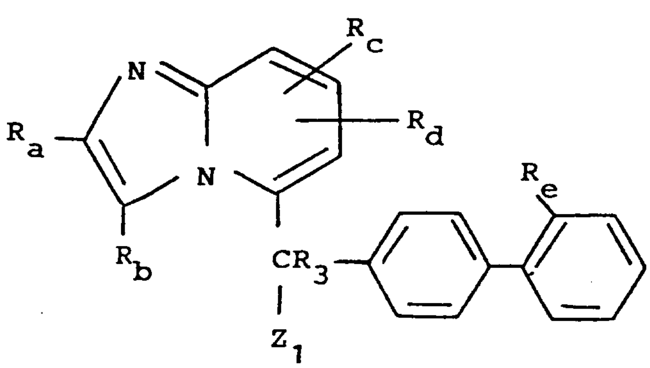

,(VI)

in der

$R_a$ bis $R_e$ und $R_3$ wie eingangs definiert sind und

$Z_1$ eine durch ein Hydrid austauschbare Gruppe, z. B. ein Chlor-, Brom- oder Jodatom, eine Hydroxy-, p-Toluolsulfonyloxy-, $R_8$-CO- oder $R_8$-CS-Gruppe, wobei $R_8$ eine Phenyl-, Phenyloxy-, Phenylthio- oder eine Alkoxygruppe mit 1 bis 3 Kohlenstoffatomen bedeutet, mit einem Organometallhydrid.

Die Umsetzung erfolgt zweckmäßigerweise in einem Lösungsmittel oder Lösungsmittelgemisch wie Benzol oder Toluol, unter Verwendung eines Organometallhydrids wie Tri-n-butylzinnhydrid oder Tri-n-propylsilan bei Temperaturen zwischen 50 und 150 °C, vorzugsweise bei der Siedetemperatur des Reaktionsgemisches.

e) Zur Herstellung einer Verbindung der allgemeinen Formel I, in der X eine $-R_1N-$ oder $-(R_3CR_4)-$ Gruppe, in denen $R_1$ mit Ausnahme der Cyclopropyl-, der Cyclobutyl- und Phenylgruppe und $R_3$ wie eingangs erwähnt definiert sind und $R_4$ eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe mit 1 bis 4 Kohlenstoffatomen, wobei die Heteroarylgruppe über eine Kohlenstoff-Kohlenstoff-Bindung mit der Alkoxygruppe verknüpft ist, eine in 2-, 3- oder 4-Stellung durch eine Heteroarylgruppe substituierte Alkoxygruppe mit 2 bis 4 Kohlenstoffatomen, wobei die Heteroarylgruppe über eine Kohlenstoff-Stickstoff-Bindung mit der Alkoxygruppe verknüpft ist, eine $R_1O$- oder $R_1NR_6$-Gruppe darstellt, wobei $R_1$ und $R_3$ wie vorstehend und $R_6$ wie eingangs erwähnt definiert sind:

Umsetzung einer Verbindung der allgemeinen Formel

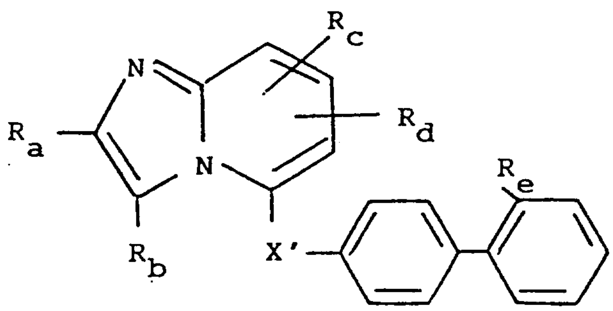

,(VII)

in der

$R_a$ bis $R_e$ wie eingangs definiert sind und

X' eine -HN- oder $-(HO-CR_3)-$ Gruppe darstellt, in welcher $R_3$ wie eingangs definiert ist, mit einer Verbindung der Formel

$R_1' - Z_2$, (VIII)

in der

$R_1'$ mit Ausnahme der Cyclopropyl-, Cyclobutyl- und Phenylgruppe die für $R_1$ eingangs erwähnten Bedeutungen besitzt und zusätzlich eine durch eine Carboxy-, Alkoxycarbonyl-, Aminocarbonyl-, Alkylaminocarbonyl-, Dialkylaminocarbonyl- oder Heteroarylgruppe substituierte Alkylgruppe mit 1 bis 4 Kohlenstoffatomen, wobei die Heteroarylgruppe über eine Kohlenstoff-Kohlenstoff-Bindung mit der Alkylgruppe verknüpft ist, eine in 2-, 3- oder 4-Stellung durch eine Heteroarylgruppe substituierte Alkylgruppe mit 2 bis 4 Kohlenstoffatomen, wobei die Heteroarylgruppe über eine Kohlenstoff-Stickstoff-Bindung mit der Alkylgruppe verknüpft ist, darstellt und

$Z_2$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Natriumhydrid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

f) Zur Herstellung einer Verbindung der allgemeinen Formel I, in der X eine $-(R_1-CO-N)-$, $-(R_1-CO-O-CR_3)-$, $-(R_1O-CO-O-CR_3)-$, $-(R_1-SO_2-O-CR_3)-$, $-(R_1-CO-NR_7-CR_3)-$, $-(R_1O-CO-NR_5-CR_3)-$ oder $-(R_1SO_2-$

$NR_7$-$CR_3$)- Gruppe darstellt:
Acylierung einer Verbindung der Formel

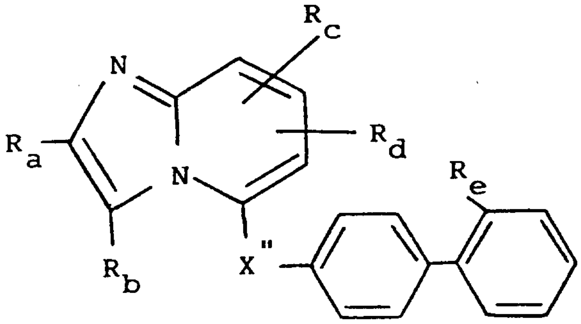

, (IX)

in der
X" eine -HN-, -(HO-$CR_3$)- oder -($R_5$NH-$CR_3$)- Gruppe darstellt, in denen $R_3$ und $R_5$ wie eingangs erwähnt definiert sind, mit einer Verbindung der Formel

$Z_3$ - U - E, (X)

in der
$Z_3$ eine nukleophile Austrittsgruppe,
U eine Carbonyl- oder Sulfonylgruppe und
E die für $R_1$ eingangs erwähnten Bedeutungen besitzt oder E zusammen mit $R_5$ eine n-Alkylengruppe mit 3 bis 5 Kohlenstoffatomen oder auch, wenn U eine Carbonylgruppe darstellt, eine $R_1$O-Gruppe, in der $R_1$ wie eingangs definiert ist, darstellen, oder mit deren reaktionsfähigen Derivaten wie deren Säurehalogeniden, Säureestern oder Säureanhydriden.

Als reaktionsfähige Derivate einer Verbindung der Formel X kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide und deren Orthoester in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid oder in einem Überschuß des Acylierungsmittels als Lösungsmittel mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100 °C, vorzugsweise jedoch bei Temperaturen zwischen -10 und 80 °C, durchgeführt.

g) Zur Herstellung einer Verbindung der allgemeinen Formel I, in der X eine -($R_5$$NR_6$-CO-O-$CR_3$)- oder -($R_5$$NR_6$-CO-$NR_5$-$CR_3$)-Gruppe darstellt:
Umsetzung einer Verbindung der allgemeinen Formel

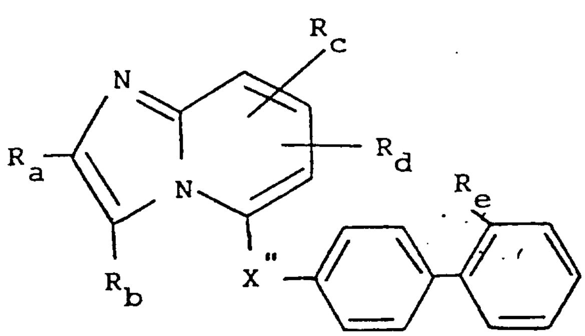

, (IX)

in der
$R_a$ bis $R_e$ und $R_3$ wie eingangs definiert sind und X" eine -(HO-$CR_3$)- oder -($R_5$NH-$CR_3$)- Gruppe

darstellt, wobei $R_3$ und $R_5$ wie eingangs definiert sind, mit einer gegebenenfalls im Reaktionsgemisch hergestellten Verbindung der allgemeinen Formel

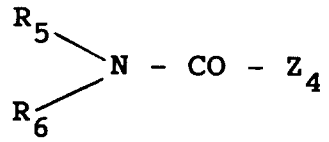

,(XI)

in der
$R_5$ und $R_6$ wie eingangs erwähnt definiert sind und
$Z_4$ eine nukleophile Austrittsgruppe wie ein Chlor- oder Bromatom oder auch
$Z_4$ und $R_5$ zusammen eine Stickstoff-Kohlenstoff-Bindung darstellen.

Die Umsetzung wird zweckmäßigerweise in einem Lösungsmittel oder Lösungsmittelgemisch wie Dichlormethan, Chloroform, Diethylether, Tetrahydrofuran, Dioxan, Dimethylformamid, Dimethylsulfoxid, Pyridin, Benzol oder Toluol bei Temperaturen zwischen 0 und 150 °C, vorzugsweise jedoch bei Temperaturen zwischen 50 und 120 °C durchgeführt.

h) Zur Herstellung einer Verbindung der allgemeinen Formel I, in der $R_e$ eine 1H-Tetrazolylgruppe darstellt:
Abspaltung eines Schutzrestes von einer Verbindung der allgemeinen Formel

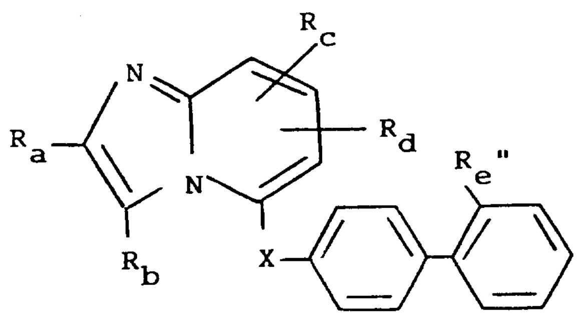

,(XII)

in der
$R_a$ bis $R_d$ und X wie eingangs definiert sind und
$R_e$'' eine in 1- oder 2-Stellung durch einen Schutzrest geschützte 1H-Tetrazolylgruppe darstellt.

Als Schutzrest kommt beispielsweise die β-Cyanoethyl-, Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100 °C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150 °C, vorzugsweise bei Temperaturen zwischen 120 und 140 °C.

i) Zur Herstellung einer Verbindung der allgemeinen Formel I, in der X ein Sauerstoffatom oder eine $-R_5N-$ Gruppe darstellt:
Umsetzung eines Imidazo[1,2-a]pyridins der allgemeinen Formel

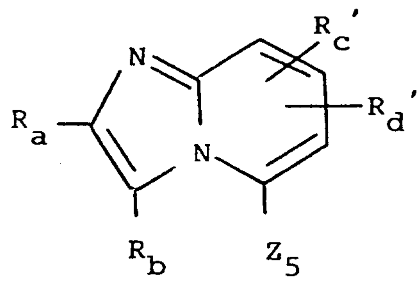

,(XIII)

in der
$R_a$ und $R_b$ wie eingangs definiert sind,
$R_c$' ein Wasserstoffatom oder eine Alkylgruppe,
$R_d$' ein Wasserstoffatom und
$Z_5$ eine nukleophile Austrittsgruppe wie ein Chlor- oder Bromatom darstellt, mit einer Biphenylverbindung der allgemeinen Formel

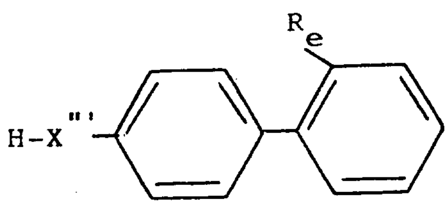

,(XIV)

in der
$R_e$ wie eingangs erwähnt definiert ist und
X''' ein Sauerstoffatom oder eine $-R_5N-$ Gruppe, in der $R_5$ wie eingangs definiert ist, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Natriumhydrid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100 °C, z.B. bei Temperaturen zwischen Raumtemperatur und 50 °C, durchgeführt.

j) Zur Herstellung einer Verbindung der allgemeinen Formel I, in der $R_e$ eine 1H-Tetrazolylgruppe darstellt:
Umsetzung einer Verbindung der allgemeinen Formel

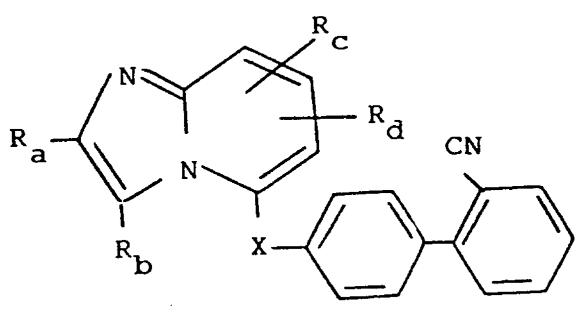

,(XV)

in der
$R_a$ bis $R_d$ und X wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150 °C, vorzugsweise bei 125 °C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

Erhält man erfindungsgemäß eine Verbindung der Formel I, in der $R_b$ ein Wasserstoffatom darstellt, so kann diese mittels Nitrierung in eine entsprechende Verbindung der Formel I, in der $R_b$ eine Nitrogruppe darstellt, übergeführt werden oder

eine Verbindung der Formel I, in der $R_b$ eine Nitrogruppe darstellt, so kann diese nach Reduktion in eine entsprechende Aminoverbindung der Formel I über die entsprechenden Diazoniumsalze in eine Verbindung der Formel I, in der $R_b$ ein Wasserstoff-, Fluor-, Chlor- oder Bromatom darstellt, übergeführt werden oder

eine Verbindung der Formel I, in der $R_b$ ein Wasserstoffatom darstellt, so kann diese mittels Halogenierung in eine entsprechende Verbindung der Formel I, in der $R_b$ ein Chlor- oder Bromatom darstellt, übergeführt werden oder

eine Verbindung der Formel I, in der $R_b$ ein Wasserstoffatom darstellt, so kann diese durch Umsetzung mit disubstituierten Formamiden, z. B. mit Dimethylformamid oder n-Methylformanilid, und Phosphoroxychlorid entsprechend einer Vilsmeier-Haack-Reaktion in eine Verbindung der Formel I, in der $R_b$ eine Formylgruppe darstellt, übergeführt werden oder

eine Verbindung der Formel I, in der $R_b$ eine Formylgruppe darstellt, so kann diese mittels Oxidation in eine Verbindung der Formel I, in der $R_b$ eine Carboxygruppe darstellt übergeführt werden oder

eine Verbindung der Formel I, in der $R_b$ eine Carboxygruppe darstellt, so kann diese durch Veresterung in eine Verbindung der Formel I, in der $R_b$ eine Alkoxycarbonylgruppe darstellt, übergeführt werden oder

eine Verbindung der Formel I, in der $R_b$ eine Formylgruppe darstellt, so kann diese durch Umsetzung mit Hydroxylamin in ein entsprechendes Aldoxim der Formel I und durch anschließende Umsetzung des Aldoxims mit einem wasserentziehenden Mittel in eine Verbindung der Formel I, in der $R_b$ eine Cyanogruppe darstellt, übergeführt werden oder

eine Verbindung der Formel I, in der $R_b$ ein Wasserstoffatom darstellt, so kann diese mittels Umsetzung mit Ammoniak oder einem primären oder sekundären Amin und Formaldehyd im Sinne einer Mannich-Reaktion in eine Verbindung der Formel I, in der $R_b$ eine $NH_2CH_2$-, $R_1NHCH_2$- oder $R_1NR_2CH_2$-Gruppe darstellt, wobei $R_1$ und $R_2$ wie eingangs erwähnt definiert sind, übergeführt werden oder

eine Verbindung der Formel I, in der X eine -($R_1SO_2O$-$CR_3$)-Gruppe darstellt, so kann diese mittels Umsetzung mit Natriumazid in die entsprechende Azidoverbindung der Formel I und gegebenenfalls durch anschließende Reduktion in die entsprechende Aminoverbindung der Formel I übergeführt werden oder

eine Verbindung der Formel I, in der X eine Carbonylgruppe darstellt, so kann diese mittels Umsetzung mit Hydroxylamin in die entsprechende Verbindung der Formel I, in der X eine -(HON=C)- Gruppe darstellt, und gegebenenfalls durch anschließende Reduktion in die entsprechende Aminoverbindung der Formel I übergeführt werden oder

eine Verbindung der Formel I, in der X eine Carbonylgruppe darstellt, so kann diese mittels Umsetzung mit 1,2-Ethandiol oder 1,3-Propandiol in die entsprechende Verbindung der Formel I, in der X eine -($R_3CR_4$)-Gruppe darstellt, wobei $R_3$ und $R_4$ zusammen eine 1,2-Ethylendioxy- oder 1,3-n-Propylendioxygruppe darstellen, übergeführt werden.

Die nachträgliche Nitrierung wird unter Verwendung von konzentrierter bis halbkonzentrierter Salpetersäure in Wasser, Eisessig oder Acetanhydrid, unter Verwendung eines Gemisches aus konzentrierter Salpetersäure und konzentrierter Schwefelsäure, mit Distickstoffpentoxid in Tetrachlorkohlenstoff und in Gegenwart von Phosphorpentoxid, mit Ethylnitrat, gegebenenfalls in Gegenwart einer Lewis- oder Brönstedt-Säure, mit Natriumnitrit in Gegenwart von Trifluoressigsäure oder unter Verwendung eines Nitroniumsalzes, z. B. Nitroniumtetrafluoroborat oder Nitroniumtrifluormethansulfonat, bei Temperaturen zwischen 0 und 100 °C, vorzugsweise bei Temperaturen zwischen 20 und 50 °C, durchgeführt.

Die nachträgliche Reduktion der Nitrogruppe wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 80 °C, vorzugsweise jedoch bei Temperaturen zwischen 20 und 40 °C, durchgeführt.

Die nachträgliche Umsetzung eines Diazoniumsalzes, z. B. des Tetrafluoroborats, des Hydrosulfates in Schwefelsäure, des Hydrochlorids oder des Hydrojodids, erforderlichenfalls in Gegenwart von Kupfer oder eines entsprechenden Kupfer-(I)-Salzes wie Kupfer-(I)-chlorid/Salzsäure oder Kupfer-(I)-Bromid/Bromwasserstoffsäure, wird bei leicht erhöhten Temperaturen, z. B. bei Temperaturen zwischen 15 und 100 °C, durchgeführt, wobei die nachträgliche Umsetzung eines Tetrafluoroborats auch durch Erwärmen des zuvor isolierten und getrockneten Diazoniumsalzes oder durch Erwärmung des Diazoniumsalzes in einem inerten Lösungsmittel, z. B. in Dichlormethan, n-Hexan, Benzol oder Toluol, erfolgen kann. Die nachträgliche Umsetzung mit unterphosphoriger Säure wird vorzugsweise bei -5 bis 0 °C durchgeführt. Das hierzu erforderliche Diazoniumsalz wird zweckmäßigerweise in einem geeigneten Lösungsmittel, z. B. in Wasser/Salzsäure, Methanol/Salzsäure, Ethanol/Salzsäure oder Dioxan/Salzsäure, durch Diazotierung einer entsprechenden Aminoverbindung mit einem Nitrit, z. B. Natriumnitrit oder einem Ester der salpetrigen Säure, bei niedrigen Temperaturen, z. B. bei Temperaturen zwischen -10 und 5 °C, hergestellt.

Die nachträgliche Halogenierung erfolgt unter Einwirkung von elementarem Chlor oder Brom, gegebenenfalls unter Zusatz von Eisen-Pulver oder einer geeigneten Lewis-Säure, z. B. Eisen-III-chlorid, Eisen-III-bromid, Aluminiumtrichlorid, Zinkchlorid oder Kupfer-II-chlorid, vorzugsweise in einem inerten Lösungsmittel wie Chloroform oder Tetrachlorkohlenstoff bei Temperaturen zwischen 0 und 80 °C, vorzugsweise bei Temperaturen zwischen 20 und 50 °C.

Die nachträgliche Formylierung erfolgt durch Umsetzung mit einem Vilsmeier-Reagens, welches vorzugsweise im Reaktionsgemisch durch Umsetzung von Dimethylformamid oder N-Methylformanilid und Phosphoroxychlorid hergestellt wird, wobei zweckmäßigerweise das eingesetzte Formamid gleichzeitig als Lösungsmittel dient, bei Temperaturen zwischen 0 und 80 °C, vorzugsweise bei Temperaturen zwischen 20 und 50 °C.

Die nachträgliche Oxidation wird vorzugsweise in einem Lösungsmittel oder Lösungsmittelgemisch, z. B. in Aceton, Pyridin, Wasser/Pyridin, Eisessig, Dichlormethan oder Chloroform bei Temperaturen zwischen -20 und 100 °C durchgeführt. Als Oxidationsmittel kommen z. B. Chromsäure in Eisessig oder in Aceton, Mangandioxid in Chloroform oder Kaliumpermanganat in Eisessig, Pyridin oder in Aceton in Betracht.

Die nachträgliche Veresterung wird zweckmäßigerweise in einem geeigneten Lösungsmittel, z. B. in einem entsprechenden Alkohol, Pyridin, Toluol, Methylenchlorid, Tetrahydrofuran oder Dioxan, in Gegenwart eines säureaktivierenden und/oder wasserentziehenden Mittels wie Thionylchlorid, Chlorameisensäureethylester, Carbonyldiimidazol oder N,N'-Dicyclohexylcarbodiimid oder dessen Isoharnstoffethern, gegebenenfalls in Gegenwart eines Reaktionsbeschleunigers wie Kupferchlorid, oder durch Umesterung, z. B. mit einem entsprechenden Kohlensäurediester, bei Temperaturen zwischen 0 und 100 °C, vorzugsweise jedoch bei Temperaturen zwischen 20 °C und der Siedetemperatur des betreffenden Lösungsmittels, durchgeführt.

Die nachträgliche Oximbildung erfolgt vorzugsweise in einem Lösungsmittel wie Methanol, Ethanol, Dichlormethan, Tetrahydrofuran, Dioxan, Benzol oder Toluol durch Umsetzung mit Hydroxylamin, zweckmäßigerweise in Gegenwart einer katalytischen Menge einer Säure wie Salzsäure oder Schwefelsäure bei Temperaturen zwischen 0 und 50 °C, vorzugsweise bei Temperaturen zwischen 10 und 30 °C.

Die nachträgliche Dehydratisierung einer entsprechenden Oximverbindung wird mit einem wasserentziehenden Mittel wie Phosphorpentoxid, Schwefelsäure oder p-Toluolsulfonsäurechlorid gegebenenfalls in einem Lösungsmittel wie Methylenchlorid oder Pyridin bei Temperaturen zwischen 0 und 100 °C, vorzugsweise bei Temperaturen zwischen 20 und 80 °C, durchgeführt.

Die nachträgliche Umsetzung mit Ammoniak oder einem primären oder sekundären Amin und Formaldehyd im Sinne einer Mannich-Reaktion wird zweckmäßigerweise in einem geeigneten Lösungsmittel oder Lösungsmittelgemisch wie Wasser, Methanol, Ethanol, Ethanol/Wasser oder Dioxan, gegebenenfalls unter Zusatz von Essigsäure, bei einer Temperatur zwischen 0 und 80 °C, vorzugsweise bei Temperaturen zwischen 20 und 50 °C, durchgeführt.

Die nachträgliche Herstellung eines Azids wird vorzugsweise in einem Lösungsmittel wie Wasser/Methanol, Wasser/Aceton, Ethanol, Tetrahydrofuran oder Dioxan durch Umsetzung eines entsprechenden Sulfonsäureesters mit einem Alkaliazid wie Natriumazid bei Temperaturen zwischen 0 und 50 °C, vorzugsweise bei Raumtemperatur, durchgeführt.

Die nachträgliche Dioxolan- oder Dioxanbildung wird in Gegenwart eines entsprechenden 1,2- oder 1,3-Dihydroxyalkans, welches zweckmäßigerweise gleichzeitig als Lösungsmittel dient, zweckmäßigerweise in Gegenwart einer katalytischen Menge einer Säure wie Schwefelsäure oder p-Toluonsulfonsäure bei Temperaturen zwischen 20 und 100 °C, vorzugsweise zwischen 30 und 80 °C, durchgeführt.

Bei den vorstehend beschriebenen Umsetzungen können gegebenenfalls vorhandene reaktive Gruppen wie Hydroxy-, Amino-, Alkylamino- oder Formylgruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe, als Schutzrest für eine Amino-, Alkylamino- oder Iminogruppe die Acetyl-, Benzoyl-, Ethoxycarbonyl-, Phthalyl- oder Benzylgruppe und als Schutzrest für eine Formylgruppe eine Acetal- oder Thioacetalgruppe, z. B. die 1,2-Ethylendioxy, 1,3-n-Propylendioxy-, 1,2-Ethylendithio- oder 1,3-n-Propylendithiogruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100 °C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 °C, vorzugsweise jedoch bei Raumtemperatur, und einem Wasserstoffdruck von 1 bis 7 bar, vorzugsweise jedoch von 3 bis 5 bar.

Ferner können die erhaltenen Verbindungen der allgemeinen Formel I mit mindestens einem optisch aktiven Kohlenstoffatom in ihre Enantiomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XV sind teilweise literaturbekannt oder man erhält diese nach literaturbekannten Verfahren.

So erhält man beispielsweise eine Verbindung der allgemeinen Formel II, in der $R_a$ eine geradkettige oder verzweigte Alkylgruppe mit 1 bis 6 Kohlenstoffatomen, eine Cycloalkylgruppe oder eine durch eine Alkoxygruppe substituierte Alkylgruppe und $R_b'$ ein Wasserstoffatom oder eine Alkylgruppe darstellt, oder die in 3-Position durch eine Alkoxycarbonylgruppe substituiert ist, durch Umsetzung eines entsprechenden 2-Aminopyridins der allgemeinen Formel

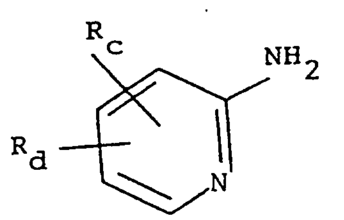

,(XVI)

in der
$R_c$ und $R_d$ wie eingangs erwähnt definiert sind, mit einer Carbonylverbindung der Formel

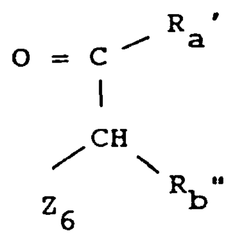

,(XVII)

in der
$R_a'$ eine geradkettige oder verzweigte Alkylgruppe mit 1 bis 6 Kohlenstoffatomen, eine Cycloalkylgruppe oder eine durch eine Alkoxygruppe substituierte Alkylgruppe darstellt, $R_b''$ ein Wasserstoffatom, eine Alkylgruppe oder eine Alkoxycarbonylgruppe darstellt und
$Z_6$ eine nucleophile Austrittsgruppe wie ein Halogenatom, z. B. ein Chlor-, Brom- oder Jodatom, oder eine substituierte Sulfonyloxygruppe, z. B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Ethanol, Isopropanol, Benzol, Glycol, Glycolmonomethylether, 1,2-Dimethoxyethan, Methylenchlorid, Diethylether, Tetrahydrofuran, Dioxan, Dimethylsulfoxid oder Dimethylformamid gegebenenfalls in Gegenwart eines säurebindenden Mittels, wie Natriumcarbonat, Kaliumcarbonat, Triethylamin oder Pyridin, wobei die beiden letzteren auch als Lösungsmittel verwendet werden können, beispielsweise bei Temperaturen zwischen 0 und 150°C, vorzugsweise bei Temperaturen zwischen 20 und 100°C. Die Umsetzung kann jedoch auch ohne Lösungsmittel durchgeführt werden.

Eine Verbindung der Formel II, in der $R_a$ eine Alkoxygruppe darstellt und die in 3-Position durch eine Alkoxycarbonylgruppe substituiert ist, erhält man beispielsweise analog dem in der EP-A-0,383,319 beschriebenen Weg durch Umsetzung eines 2-Aminopyridins der allgemeinen Formel XVI mit Chloressigsäure, anschließende alkalische Hydrolyse und Chlorierung mit Phosphoroxychlorid zum entsprechenden 2-Chlor-imidazo[1,2-a]-pyridin-Derivat, Einführung der Ethoxycarbonylgruppe in 3-Position durch Metallierung mit n-Butyllithium und Umsetzung der Aryllithiumverbindung mit Chlorameisensäureethylester und anschließende Substitution des Chloratoms in 2-Position durch eine Alkoxygruppe mittels Reaktion mit einem entsprechenden Natriumalkoholat, gegebenenfalls unter gleichzeitiger Umesterung der Ethoxycarbonylgruppe in eine entsprechende Alkoxycarbonylgruppe.

Eine Verbindung der Formel II, in der $R_a$ eine Alkoxygruppe und $R_b'$ ein Wasserstoffatom darstellt, erhält man durch Verseifung und anschließende Decarboxylierung einer Verbindung der Formel II, in der $R_a$ eine Alkoxygruppe darstellt und die in 3-Position durch eine Alkoxycarbonylgruppe substituiert ist.

Eine Verbindung der Formel II, in der $R_b'$ eine Trifluormethylgruppe darstellt, erhält man durch Umsetzung einer Verbindung der Formel II, die in 3-Position durch eine Carboxy- oder Alkoxycarbonylgruppe substituiert ist, mit einem Fluorierungsmittel, z. B. mit Diethylaminoschwefeltrifluorid, Perchlorylfluorid oder Molybdänhexafluorid.

Eine Verbindung der Formel II, in der $R_b'$ eine Hydroxymethylgruppe darstellt, erhält man durch Reduktion einer Verbindung der Formel II, die in 3-Position durch eine Alkoxycarbonylgruppe substituiert ist, z. B. mit Lithiumaluminiumhydrid, Lithiumborhydrid, Diisobutylaluminiumhydrid, Lithiumtriethylborhydrid oder Triethoxysilan.

Eine Verbindung der Formel II, in der $R_a$ eine Alkoxygruppe und $R_b'$ eine Alkylgruppe darstellt, erhält man durch Reduktion einer entsprechenden Carbonylverbindung der Formel II, in der $R_a$ eine Alkoxygruppe darstellt.

Die als Ausgangsstoffe verwendeten Verbindungen der allgemeinen Formeln IV, V, VI, VII, IX, XII und XV erhält man vorzugsweise durch Umsetzung eines entsprechenden Imidazo[1,2-a]pyridins mit einer entsprechenden Biphenylcarbonylverbindung. Eine so erhaltene Hydroxymethylenverbindung wird anschließend durch Acylierung, Alkylierung, Azidbildung und anschließende Reduktion in die gewünschte Verbindung übergeführt.

Die als Ausgangsstoffe verwendeten Verbindungen der allgemeinen Formel XIII werden durch Umsetzung eines geeigneten substituierten Aminopyridins mit einem entsprechend substituierten $\beta$-Ketocarbonylverbindung erhalten.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = (R,S)-2-Ethyl-8-methyl-5-[α-(2'-carboxybiphenyl-4-yl)-α-hydroxy-methyl]imidazo[1,2-a]pyridin,
B = (R,S)-2-Ethyl-8-methyl-5-[α-(2'-carboxybiphenyl-4-yl)-α-acetoxy-methyl]imidazo[1,2-a]pyridin,
C = (R,S)-2-Ethyl-8-methyl-5-[α-(2'-carboxybiphenyl-4-yl)-α-cyclohexylaminocarbonyloxy-methyl]-imidazo[1,2-a]pyridin und
D = (R,S)-2-n-Propyl-8-methyl-5-[α-(2'-carboxybiphenyl-4-yl)-α-hydroxy-methyl]imidazo[1,2-a]pyridin-hydrat

auf ihre biologischen Wirkungen wie folgt untersucht:

Methodenbeschreibung Angiotensin II-Rezeptorbindung

Das Gewebe (Rattenlunge) wird in Tris-Puffer (50 mMol Tris, 150 mMol NaCl, 5 mMol EDTA, pH 7.40) homogenisiert und zweimal je 20 Min. bei 20.000 x g zentrifugiert. Das endgültige Pellet wird in Inkubations-Puffer (50 mMol Tris, 5 mMol $MgCl_2$, 0,2 % BSA, pH 7,40) 1:75, bezogen auf das Feuchtgewicht des Gewebes, resuspendiert. Je 0,1 ml Homogenat wird für 60 Min. bei 37°C mit 50 pM [$^{125}$I]-Angiotensin II (NEN, Dreieich, FRG) und steigenden Konzentrationen der Testsubstanz in einem Gesamtvolumen von 0,25 ml inkubiert. Die Inkubation wird durch rasche Filtration durch Glasfiber-Filtermatten beendet. Die Filter werden je 4 ml eiskaltem Puffer (25 mMol Tris, 2.5 mMol $MgCl_2$, 0,1 % BSA, pH 7,40) gewaschen. Die gebundene Radioaktivität wird in einem Gamma-Counter ermittelt. Aus der Dosis-Wirkungskurve wird der entsprechende $IC_{50}$-Wert ermittelt.

Die Substanzen A bis D zeigen in dem beschriebenen Test folgende $IC_{50}$-Werte:

| Substanz | $IC_{50}$ [nM] |
|---|---|
| A | 46 |
| B | 30 |
| C | 230 |
| D | 420 |

Zusätzlich wurden die Verbindungen A bis D an wachen, renal hypertensiven Ratten auf ihre Wirkung nach oraler Gabe nach literaturbekannten Methoden getestet. Bei einer Dosis von 10 mg/kg zeigten diese Verbindungen eine blutdrucksenkende Wirkung.

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften, nämlich einer blutdrucksenkenden Wirkung mit einer diuretischen/saluretischen Komponente, eignen sich die neuen Verbindungen und deren physiologisch verträgliche Salze zur Behandlung der Hypertonie und Herzinsuffizienz, ferner zur Behandlung der chronischen Niereninsuffizienz, zur Behandlung ischämischer peripherer (beispielsweise Raynaud's Syndrom) sowie cerebrovaskulärer Durchblutungsstörungen, der myokardialen Ischämie (Angina), zur Prävention der Herzinsuffizienzprogression nach Myokard-Infarkt, zur Behandlung der diabetischen Nephro- und Retinopathie, des Glaukoms, von gastrointestinalen Erkrankungen und Blasenerkrankungen.

Weiterhin eignen sich die neuen Verbindungen und deren physiologisch verträgliche Salze zur Behandlung pulmonaler Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose, der diabetischen Angiopathie, der Hyperuricämie und der Gicht. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, sowie von Störungen kognitiver Funktionen.

Die zur Erzielung einer entsprechenden Wirkung am Erwachsenen erforderliche Dosierung beträgt zweckmäßigerweise bei intravenöser Gabe 0,5 bis 100 mg, vorzugsweise 1 bis 70 mg, und bei oraler Gabe 0,1 bis 200 mg, vorzugsweise 1 bis 100 mg, jeweils 1 bis 3 x täglich. Hierzu lassen sich die erfindungsgemäß hergestellten Verbindungen der allgemeinen Formel I, gegebenenfalls in Kombination mit anderen

Wirksubstanzen wie z.B. Blutdrucksenker, ACE-Hemmer, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin, Nitrendipin, Captopril, Enalapril, Lisinopril, Cilazapril, Quinapril, Fosinopril und Ramipril in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

Beispiel 1

(R,S)-2-Ethyl-8-methyl-5-[α-(2'-carboxybiphenyl-4-yl)-α-hydroxy-methyl]imidazo[1,2-a]pyridin

a) 2-Ethyl-8-methyl-imidazo[1,2-a]pyridin

21,6 g (0,2 mol) 2-Amino-3-picolin werden in 300 ml 1,2-Dimethoxyethan gelöst und mit 33,2 g (0,2 Mol) 1-Methansulfonyloxy-2-butanon versetzt. Die Reaktionsmischung wird 3 Tage bei Raumtemperatur gerührt. Danach wird das Solvens im Vakuum abgedampft. Der Rückstand wird über eine Kieselgelsäule (Korngröße: 0,063 bis 0,02 mm) gereinigt, wobei als Elutionsmittel anfangs Methylenchlorid, danach Gemische von Methylenchlorid und Ethanol mit steigender Polarität (2-4% Ethanol) verwendet werden. Die einheitlichen Fraktionen werden im Vakuum eingeengt.
Ausbeute: 7,8 g (24 % der Theorie),
Öl, $R_f$-Wert: 0,50 (Kieselgel; Laufmittel: Methylenchlorid/Ethanol = 9:1).

b) (R,S)-2-Ethyl-8-methyl-5-[α-(2'-(tert.butyloxycarbonyl)-biphenyl-4-yl)-α-hydroxy-methyl]imidazo[1,2-a]-pyridin

2,30 g (16 mMol) 2-Ethyl-8-methyl-imidazo[1,2-a]pyridin werden in 30 ml absolutem Tetrahydrofuran gelöst und bei -78°C unter Stickstoff langsam mit 10,4 ml einer 1,1 molaren Lösung von n-Butyllithium in Hexan (16,6 mMol) versetzt. Die Reaktionsmischung wird 30 Minuten bei -78°C gerührt und danach langsam auf -20°C erwärmt. Anschließend wird erneut auf -78°C abgekühlt und eine Lösung von 4,7 g (16,6 mMol) 4-(2'-tert.Butyloxycarbonyl)phenyl-benzaldehyd in 15 ml absolutem Tetrahydrofuran innerhalb von 30 Minuten zugetropft. Die Mischung wird 3 Stunden bei -78°C und anschließend 12 Stunden bei Raumtemperatur gerührt. Nach Zugabe von 30 ml gesättigter Ammoniumchloridlösung wird 4 x mit je 50 ml Essigester extrahiert. Die vereinigten organischen Extrakte werden mit gesättigter Kochsalzlösung gewaschen, über Magnesiumsulfat getrocknet und eingedampft. Der Rückstand wird über eine Kieselgelsäule (Korngröße: 0,063-0,02 mm) gereinigt, wobei als Elutionsmittel anfangs Methylenchlorid, danach Gemische von Methylenchlorid und Ethanol mit steigender Polarität (2 bis 5% Ethanol) verwendet werden. Die einheitlichen Fraktionen werden im Vakuum eingeengt.
Ausbeute: 3,70 g (52 % der Theorie),
Öl, $R_f$-Wert: 0,50 (Kieselgel; Laufmittel: Methylenchlorid/Ethanol = 95:5).

c) (R,S)-2-Ethyl-8-methyl-5-[α-(2'-carboxybiphenyl-4-yl)-α-hydroxy-methyl]imidazo[1,2-a]pyridin

3,50 g (7,9 mMol) (R,S)-2-Ethyl-8-methyl-5-[α-(2'-(tert.-butyloxycarbonyl)biphenyl-4-yl)-α-hydroxy-methyl]imidazo-[1,2-a]pyridin werden in 25 ml Methylenchlorid gelöst und mit 5 ml Trifluoressigsäure versetzt. Die Mischung wird 12 Stunden bei Raumtemperatur gerührt und anschließend eingedampft. Der Rückstand wird mit 25 ml Wasser versetzt und durch Zugabe von konz. Ammoniak in Lösung gebracht. Der nach Zugabe von Eisessig gebildete Niederschlag wird abgesaugt, mit Wasser gewaschen und bei 60°C

im Vakuum getrocknet.
Ausbeute: 0,49 g (16 % der Theorie),
Schmelzpunkt: > 250 ° C

| $C_{24}H_{22}N_2O_3$ (386,46) | | | |
|---|---|---|---|
| Ber.: | C 74,59 | H 5,74 | N 7,25 |
| Gef.: | 74,08 | 5,84 | 7,59 |

Massenspektrum: $M^+$ = 386.

Beispiel 2

(R,S)-2-Ethyl-5-[α-(2'-carboxybiphenyl-4-yl)-α-hydroxymethyl]imidazo[1,2-a]pyridin

a) 2-Ethyl-imidazo[1,2-a]pyridin

Hergestellt analog Beispiel 1a aus 2-Amino-pyridin und 1-Methansulfonyloxy-2-butanon.
Ausbeute: 34 % der Theorie,
Öl, $R_f$-Wert: 0,30 (Kieselgel; Laufmittel: Methylenchlorid/Ethanol = 95:5).

b) (R,S)-2-Ethyl-5-[α-(2'-(tert.butyloxycarbonyl)biphenyl-4-yl)-α-hydroxy-methyl]imidazo[1,2-a]pyridin

Hergestellt analog Beispiel 1b aus 2-Ethyl-imidazo[1,2-a]-pyridin und 4-(2'-tert.Butyloxycarbonyl)phenyl-benzaldehyd.
Ausbeute: 23 % der Theorie,
Öl, $R_f$-Wert: 0,15 (Kieselgel; Laufmittel: Methylenchlorid/Ethanol = 95:5).

c) (R,S)-2-Ethyl-5-[α-(2'-carboxybiphenyl-4-yl)-α-hydroxy-methyl]imidazo[1,2-a]pyridin

Hergestellt analog Beispiel 1c aus (R,S)-2-Ethyl-5-[α-(2'-(tert.butyloxycarbonyl)biphenyl-4-yl)-α-hydroxy-methyl]imidazo[1,2-a]pyridin und Trifluoressigsäure.
Ausbeute: 38 % der Theorie,
Schmelzpunkt: 235 ° C (Zersetzung)

| $C_{23}H_{20}N_2O_3$ (372,43) | | | |
|---|---|---|---|
| Ber.: | C 74,18 | H 5,41 | N 7,52 |
| Gef.: | 74,21 | 5,41 | 7,18 |

Massenspektrum: $M^+$ = 372.

Beispiel 3

2-Ethyl-5-[(2'-carboxybiphenyl-4-yl)carbonyl]imidazo[1,2-a]-pyridin

a) 2-Ethyl-5-[(2'-(tert.butyloxycarbonyl)biphenyl-4-yl)carbonyl]imidazo[1,2-a]pyridin

600 mg (1,4 mMol) (R,S)-2-Ethyl-5-[α-(2'-(tert.butyloxycarbonyl)biphenyl-4-yl)-α-hydroxy-methyl]-imidazo[1,2-a]pyridin werden in 30 ml Chloroform gelöst und mit 2 g Mangandioxid versetzt. Die Mischung wird 24 Stunden bei Raumtemperatur gerührt. Anschließend wird filtriert, das Filtrat mit Methylenchlorid gewaschen, und die vereinigten organischen Phasen werden eingedampft. Der Rückstand wird über eine Kieselgelsäule (Korngröße: 0,063 bis 0,02 mm) gereinigt, wobei als Elutionsmittel anfangs Methylenchlorid, danach Methylenchlorid mit 2,5% Ethanol verwendet wird. Die einheitlichen Fraktionen werden im Vakuum eingeengt.
Ausbeute: 0,35 g (59 % der Theorie),
Öl, $R_f$-Wert: 0,75 (Kieselgel; Laufmittel: Methylenchlorid/Ethanol = 9:1).

b) 2-Ethyl-5-[(2'-carboxybiphenyl-4-yl)carbonyl]imidazo-[1,2-a]pyridin

Hergestellt analog Beispiel 1c aus 2-Ethyl-5-[(2'-(tert.butyloxycarbonyl)biphenyl-4-yl)carbonyl]imidazo-[1,2-a]pyridin und Trifluoressigsäure.
Ausbeute: 79 % der Theorie,
Schmelzpunkt: 115 °C

| $C_{23}H_{18}N_2O_3$ (370,41) | | | |
|---|---|---|---|
| Ber.: | C 74,58 | H 4,90 | N 7,56 |
| Gef.: | 73,82 | 5,02 | 7,48 |

Massenspektrum: $M^+$ = 370.

Beispiel 4

(R,S)-2-Ethyl-5-[α-(2'-carboxybiphenyl-4-yl)-α-acetoxymethyl]imidazo[1,2-a]pyridin

a) (R,S)-2-Ethyl-5-[α-(2'-(tert.butyloxycarbonyl)biphenyl-4-yl)-α-acetoxy-methyl]imidazo[1,2-a]pyridin

350 mg (0,8 mMol) (R,S)-2-Ethyl-5-[α-(2'-(tert.butyloxycarbonyl)biphenyl-4-yl)-α-hydroxy-methyl]-imidazo[1,2-a]pyridin werden in 4 ml Acetanhydrid gelöst und 30 Minuten auf 100 °C erhitzt. Danach wird das Reaktionsgemisch eingedampft, der Rückstand wird mit 10 ml Wasser versetzt und mit Essigester extrahiert. Die vereinigten organischen Phasen werden über Magnesiumsulfat getrocket und anschließend eingedampft. Der Rückstand wird ohne Reinigung weiter umgesetzt.
Ausbeute: 0,39 g (100 % der Theorie),
Öl, $R_f$-Wert: 0,65 (Kieselgel; Laufmittel: Methylenchlorid/Ethanol = 95:5).

b) (R,S)-2-Ethyl-5-[α-(2'-carboxybiphenyl-4-yl)-α-acetoxy-methyl]imidazo[1,2-a]pyridin

Hergestellt analog Beispiel 1c aus (R,S)-2-Ethyl-5-[α-(2'-(tert.butyloxycarbonyl)biphenyl-4-yl)-α-acetoxy-methyl]-imidazo[1,2-a]pyridin und Trifluoressigsäure.
Ausbeute: 51 % der Theorie,
Schmelzpunkt: 217-218 °C

| $C_{25}H_{22}N_2O_4$ (414,47) | | | |
|---|---|---|---|
| Ber.: | C 72,45 | H 5,35 | N 6,76 |
| Gef.: | 72,23 | 5,05 | 6,89 |

Massenspektrum: $M^+$ = 414.

Beispiel 5

(R,S)-2-Ethyl-8-methyl-5-[α-(2'-carboxybiphenyl-4-yl)-α-acetoxy-methyl]imidazo[1,2-a]pyridin

a) (R,S)-2-Ethyl-8-methyl-5-[α-(2'-tert.butyloxycarbonyl)-biphenyl-4-yl)-α-acetoxy-methyl]imidazo[1,2-a]-pyridin

Hergestellt analog Beispiel 4a aus (R,S)-2-Ethyl-8-methyl-5-[α-(2'-tert.butyloxycarbonyl)biphenyl-4-yl)-α-hydroxymethyl]imidazo[1,2-a]pyridin und Acetanhydrid.
Ausbeute: 100 % der Theorie,
Öl, $R_f$-Wert: 0,60 (Kieselgel; Laufmittel: Methylenchlorid/Ethanol = 9:1).

b) (R,S)-2-Ethyl-8-methyl-5-[α-(2'-carboxybiphenyl-4-yl)-α-acetoxy-methyl]imidazo[1,2-a]pyridin

Hergestellt analog Beispiel 1c aus (R,S)-2-Ethyl-8-methyl-5-[α-(2'-tert.butyloxycarbonyl)biphenyl-4-yl)-α-acetoxy-methyl]imidazo[1,2-a]pyridin und Trifluoressigsäure.
Ausbeute: 58 % der Theorie,
Schmelzpunkt: 228-230 °C
$C_{26}H_{24}N_2O_4$ (428,49)
Massenspektrum: $M^+$ = 428.

Beispiel 6

(R,S)-2-Ethyl-8-methyl-5-[α-(2'-carboxybiphenyl-4-yl)-α-methoxy-methyl]imidazo[1,2-a]pyridin

a) (R,S)-2-Ethyl-8-methyl-5-[α-(2'-(tert.butyloxycarbonyl)-biphenyl-4-yl)-α-methoxy-methyl]imidazo[1,2-a]-pyridin

440 mg (1,0 mMol) (R,S)-2-Ethyl-8-methyl-5-[α-(2'-(tert.-butyloxycarbonyl)biphenyl-4-yl)-α-hydroxy-methyl]imidazo[1,2-a]pyridin werden in 5 ml Dimethylformamid gelöst. Nach Zugabe von 130 mg (1,2 mMol) Kalium-tert.butylat wird 30 Minuten bei Raumtemperatur gerührt. Danach werden 170 mg (1,2 mMol) Methyliodid zugegeben. Anschließend wird das Reaktionsgemisch weitere 5 Stunden bei Raumtemperatur gerührt. Nach Zugabe von 10 ml Wasser wird 2 x mit Essigester extrahiert. Die vereinigten organischen Phasen werden mit gesättigter Natriumchloridlösung gewaschen, über Magnesiumsulfat getrocknet und anschließend eingedampft. Der Rückstand wird über eine Kieselgelsäule (Korngröße: 0,063 bis 0,02 mm) gereinigt, wobei als Elutionsmittel Methylenchlorid mit 1,5% Ethanol verwendet wird. Die einheitlichen Fraktionen werden im Vakuum eingeengt.
Ausbeute: 0,13 g (29 % der Theorie),
Öl, $R_f$-Wert: 0,50 (Kieselgel; Laufmittel: Methylenchlorid/Ethanol = 95:5).

b) (R,S)-2-Ethyl-8-methyl-5-[α-(2'-carboxybiphenyl-4-yl)-α-methoxy-methyl]imidazo[1,2-a]pyridin

Hergestellt analog Beispiel 1c aus (R,S)-2-Ethyl-8-methyl-5-[α-(2'-(tert.butyloxycarbonyl)biphenyl-4-yl)-α-methoxy-methyl]imidazo[1,2-a]pyridin und Trifluoressigsäure.
Ausbeute: 89 % der Theorie,
Schmelzpunkt: 120-123 °C
$C_{25}H_{24}N_2O_3$ (400,48)
Massenspektrum: $M^+$ = 400.

Beispiel 7

2-Ethyl-8-methyl-5-[(2'-carboxybiphenyl-4-yl)methyl]imidazo[1,2-a]pyridin

a) (R,S)-2-Ethyl-8-methyl-5-[α-(2'-(tert.butyloxycarbonyl)-biphenyl-4-yl)-α-(methylthio-thiocarbonyloxy-methyl]-imidazo[1,2-a]pyridin

440 mg (1,0 mMol) (R,S)-2-Ethyl-8-methyl-5-[α-(2'-(tert.-butyloxycarbonyl)biphenyl-4-yl)-α-hydroxy-methyl]imidazo[1,2-a]pyridin werden unter Stickstoff in 10 ml absolutem Tetrahydrofuran gelöst und mit 5 mg Imidazol versetzt. Nach Zugabe von 72 mg (1,5 mMol) Natriumhydrid (50%ig) wird 20 Minuten bei Raumtemperatur gerührt. Danach werden 228 mg (3,0 mMol) Schwefelkohlenstoff und nach weiteren 30 Minuten 253 mg (1,8 mMol) Methyliodid zugegeben. Anschließend wird das Reaktionsgemisch weitere 15 Minuten bei Raumtemperatur gerührt. Nach Zugabe von 3 Tropfen Essigsäure werden 20 ml Ether zugegeben. Die organische Phase wird mit gesättigter Natriumhydrogencarbonatlösung und anschließend mit gesättigter Natriumchloridlösung gewaschen, über Magnesiumsulfat getrocknet und dann eingedampft. Der Rückstand wird ohne Reinigung weiter umgesetzt.
Ausbeute: 0,57 g,
Öl, $R_f$-Wert: 0,55 (Kieselgel; Laufmittel: Methylenchlorid/Ethanol = 95:5).

b) 2-Ethyl-8-methyl-5-[(2'-tert.butyloxycarbonyl)biphenyl-4-yl)methyl]imidazo[1,2-a]pyridin

532 mg (1,0 mMol) (R,S)-2-Ethyl-8-methyl-5-[α-(2'-(tert.-butyloxycarbonyl)biphenyl-4-yl)-α-(methylthiothiocarbonyloxy)-methyl]imidazo[1,2-a]pyridin werden in 10 ml Toluol gelöst und unter Stickstoff mit 437 mg (1,5 mMol) Tri-n-butylzinnhydrid versetzt. Die Reaktionsmischung wird 18 Stunden unter Rückfluß erhitzt. Anschließend wird im Vakuum eingedampft und der Rückstand über eine Kieselgelsäule (Korngröße: 0,063-0,02 mm) gereinigt. Dabei wird als Elutionsmittel anfangs Methylenchlorid, später Methylenchlorid mit 1 bis 2,5% Ethanol verwendet. Die einheitlichen Fraktionen werden im Vakuum eingeengt.
Ausbeute: 160 mg (38 % der Theorie),
Öl, $R_f$-Wert: 0,40 (Kieselgel; Laufmittel: Methylenchlorid/Ethanol = 95:5).

c) 2-Ethyl-8-methyl-5-[(2'-carboxybiphenyl-4-yl)methyl]-imidazo[1,2-a]pyridin

Hergestellt analog Beispiel 1c aus 2-Ethyl-8-methyl-5-[(2'-tert.butyloxycarbonyl)biphenyl-4-yl)methyl]-imidazo[1,2-a]pyridin und Trifluoressigsäure.
Ausbeute: 77 % der Theorie,
Schmelzpunkt: 250 ° C
$C_{24}H_{22}N_2O_2$ (370,46)
Massenspektrum: $M^+$ = 370.

Beispiel 8

(R,S)-2-Ethyl-8-methyl-5-[α-(2'-carboxybiphenyl-4-yl)-α-cyclohexylaminocarbonyloxy-methyl]imidazo[1,2-a]-pyridin a) (R,S)-2-Ethyl-8-methyl-5-[α-[2'-(tert.butyloxycarbonyl)-

biphenyl-4-yl]-α-cyclohexylaminocarbonyloxy-methyl]-imidazo[1,2-a]pyridin

440 mg (1,0 mMol) (R,S)-2-Ethyl-5-[α-[2'-(tert.butyloxycarbonyl)biphenyl-4-yl]-α-hydroxy-methyl]-imidazo[1,2-a]pyridin und 500 mg (3,9 mMol) Cyclohexylisocyanat werden in 5 ml absolutem Pyridin gelöst und 4 Stunden zum Rückfluß erhitzt. Danach wird das Reaktionsgemisch eingedampft. Der Rückstand wird ohne Reinigung weiter umgesetzt.

b) (R,S)-2-Ethyl-8-methyl-5-[α-(2'-carboxybiphenyl-4-yl)-α-cyclohexylaminocarbonyloxy-methyl]imidazo[1,2-a]pyridin

Hergestellt analog Beispiel 1c aus (R,S)-2-Ethyl-8-methyl-5-[α-[2'-(tert.butyloxycarbonyl)biphenyl-4-yl]-α-cyclohexylaminocarbonyloxy-methyl]imidazo[1,2-a]pyridin und Trifluoressigsäure.
Ausbeute: 33 % der Theorie,
Schmelzpunkt: 243 ° C (Zers)
$C_{31}H_{33}N_3O_4$ (511,63)
Massenspektrum: $M^+$ = 511.

Beispiel 9

(R,S)-2-Ethyl-8-methyl-5-[α-(2'-carboxybiphenyl-4-yl)-α-cyclohexylcarbonyloxy-methyl]imidazo[1,2-a]pyridin

a) (R,S)-2-Ethyl-8-methyl-5-[α-[2'-(tert.butyloxycarbonyl)-biphenyl-4-yl]-α-cyclohexylcarbonyloxy-methyl]-imidazo[1,2-a]pyridin

Hergestellt analog Beispiel 4a aus (R,S)-2-Ethyl-8-methyl-5-[α-[2'-(tert.butyloxycarbonyl)biphenyl-4-yl]-α-hydroxymethyl]imidazo[1,2-a]pyridin und Cyclohexylcarbonsäurechlorid.
Ausbeute: 100 % der Theorie,
$R_f$-Wert: 0,50 (Kieselgel; Laufmittel: Essigester/Petrolether = 1:1).

b) (R,S)-2-Ethyl-8-methyl-5-[α-(2'-carboxybiphenyl-4-yl)-α-cyclohexylcarbonyloxy-methyl]imidazo[1,2-a]-pyridin

Hergestellt analog Beispiel 1c aus (R,S)-2-Ethyl-8-methyl-5-[α-[2'-(tert.butyloxycarbonyl)biphenyl-4-yl]-α-cyclohexylcarbonyloxy-methyl]imidazo[1,2-a]pyridin und Trifluoressigsäure.
Ausbeute: 54 % der Theorie,
Schmelzpunkt: 243 °C (Zers.)
$C_{31}H_{32}N_2O_4$ (496,61)
Massenspektrum: $M^+$ = 496.

Beispiel 10

(R,S)-2-n-Propyl-8-methyl-5-[α-(2'-carboxybiphenyl-4-yl)-α-hydroxy-methyl]imidazo[1,2-a]pyridin-hydrat

a) 2-n-Propyl-3-ethoxycarbonyl-8-methyl-imidazo[1,2-a]-pyridin

2,3 g (11,9 mMol) 2-Chlor-3-oxo-hexancarbonsäure-ethylester und 2,6 g (23,8 mMol) 2-Amino-3-picolin werden in 10 ml Ethylenglykol-dimethylether gelöst und 3 Tage zum Rückfluß erhitzt. Anschließend wird das Solvens im Vakuum abgedampft und der Rückstand in Wasser/Essigester 1:1 aufgenommen. Nach der Extraktion wird die organische Phase abgetrennt, mit gesättigter Kochsalzlösung gewaschen, über Magnesiumsulfat getrocknet und eingedampft. Der Rückstand wird über eine Kieselgelsäule (Korngröße: 0,063 bis 0,02 mm) gereinigt, wobei als Elutionsmittel Gemische von Petrolether/Essigester mit steigender Polarität (85:15, 80:20 und 1:1) verwendet werden. Die einheitlichen Fraktionen werden im Vakuum eingeengt.
Ausbeute: 2,0 g (69 % der Theorie),
Schmelzpunkt: 68-70 °C.

b) 2-n-Propyl-3-carboxy-8-methyl-imidazo[1,2-a]pyridin

21,0 g (86 mMol) 2-n-Propyl-3-ethoxycarbonyl-8-methyl-imidazo[1,2-a]pyridin werden in 200 ml Ethanol gelöst, mit 100 ml 2N Natronlauge versetzt und 4 Tage bei Raumtemperatur gerührt. Das Solvens wird bei 40 °C im Vakuum abgedampft, der Rückstand wird mit Wasser verdünnt und mit 20 g Ammoniumchlorid versetzt. Der pH-Wert wird durch Zusatz von Eisessig auf 6,5 eingestellt. Nach 2 Stunden wird der ausgefallene Niederschlag abgesaugt, mit Wasser gewaschen und bei 40 °C im Vakuum getrocknet.
Ausbeute: 17,3 g (92 % der Theorie),
Schmelzpunkt: 164-165 °C.

c) 2-n-Propyl-8-methyl-imidazo[1,2-a]pyridin

17,3 g (79 mMol) 2-n-Propyl-3-carboxy-8-methyl-imidazo[1,2-a]pyridin werden in 200 ml Diethylenglykol-dimethylether gelöst und 1 Stunde auf 160 °C erhitzt. Nach Abkühlung wird die Lösung auf 1,2 l Eiswasser gegossen und mit Essigester extrahiert. Die vereinigten organischen Phasen werden mit Natriumhydrogencarbonatlösung und mit Wasser gewaschen, über Magnesiumsulfat getrocknet und im Vakuum eingedampft. Der Rückstand wird in 100 ml Ether aufgenommen und mit 50 g Kieselgel und 10 g Aktivkohle versetzt.
Anschließend wird filtriert und das Filtrat eingedampft.
Ausbeute: 13,8 g (100 % der Theorie),
Öl, $R_f$-Wert: 0,50 (Kieselgel; Laufmittel: Essigester).

d) (R,S)-2-n-Propyl-8-methyl-5-[α-(2'-(tert.-butyloxycarbonyl)biphenyl-4-yl)-α-hydroxy-methyl]imidazo[1,2-a]-pyridin

Hergestellt analog Beispiel 1b aus 2-n-Propyl-8-methyl-imidazo[1,2-a]pyridin und 4-(2'-tert.Butyloxycarbonyl)phenylbenzaldehyd.
Ausbeute: 34 % der Theorie,
$R_f$-Wert: 0,60 (Kieselgel; Laufmittel: Essigester/Ethanol = 19:1).

e) (R,S)-2-n-Propyl-8-methyl-5-[α-(2'-carboxybiphenyl-4-yl)-α-hydroxy-methyl]imidazo[1,2-a]pyridin-hydrat

Hergestellt analog Beispiel 1c aus (R,S)-2-n-Propyl-8-methyl-5-[α-[2'-(tert.butyloxycarbonyl)biphenyl-4-yl)-α-hydroxymethyl]imidazo[1,2-a]pyridin und Trifluoressigäsure.
Ausbeute: 46 % der Theorie,
Schmelzpunkt: ab 190 °C (Zers.)

| $C_{25}H_{24}N_2O_3$ x $H_2O$ (418,50) | | | |
|---|---|---|---|
| Ber.:<br>Gef.: | C 71,75<br>72,03 | H 6,26<br>6,14 | N 6,69<br>7,33 |

Massenspektrum: $M^+$ = 400.

Beispiel 11

(R,S)-2-n-Propyl-8-methyl-5-[α-(2'-carboxybiphenyl-4-yl)-α-benzyloxy-methyl]imidazo[1,2-a]pyridin-semihydrat

a) (R,S)-2-n-Propyl-8-methyl-5-[α-[2'-tert.butyloxycarbonyl)biphenyl-4-yl)-α-benzyloxy-methyl]imidazo[1,2-a]-pyridin-semihydrat

Hergestellt analog Beispiel 4a aus (R,S)-2-n-Propyl-8-methyl-5-[α-[2'-(tert.butyloxycarbonyl)biphenyl-4-yl]-α-hydroxymethyl]imidazo[1,2-a]pyridin und Benzylbromid.
Ausbeute: 33 % der Theorie,
$R_f$-Wert: 0,45 (Kieselgel; Laufmittel: Essigester/Petrolether = 1:1).

b) (R,S)-2-n-Propyl-8-methyl-5-[α-(2'-carboxybiphenyl-4-yl)-α-benzyloxy-methyl]imidazo[1,2-a]pyridin-semihydrat

Hergestellt analog Beispiel 1c aus (R,S)-2-n-Propyl-8-methyl-5-[α-[2'-(tert.butyloxycarbonyl)biphenyl-4-yl]-α-benzyloxy-methyl]imidazo[1,2-a]pyridin und Trifluoressigsäure.
Ausbeute: 80 % der Theorie,
Schmelzpunkt: 113-115 °C

| $C_{32}H_{30}N_2O_3$ x 0,5 $H_2O$ (499,62) | | | |
|---|---|---|---|
| Ber.:<br>Gef.: | C 76,93<br>77,25 | H 6,25<br>6,40 | N 5,61<br>5,67 |

Massenspektrum: $M^+$ = 490.

Beispiel 12

(R,S)-2-n-Propyl-8-methyl-5-[α-(2'-carboxybiphenyl-4-yl)-α-(2-pyridyl)-methyloxy-methyl]imidazo[1,2-a]-pyridin-semihydrat

Hergestellt analog Beispiel 1c aus (R,S)-2-n-Propyl-8-methyl-5-[α-(2'-tert.butoxycarbonyl)biphenyl-α-(2-pyridyl)-methyloxy-methyl]imidazo[1,2-a]pyridin und Trifluoressigsäure.
Ausbeute: 68 % der Theorie,
Schmelzpunkt: 182-184 °C

| $C_{31}H_{29}N_3O_3$ x 0,5 $H_2O$ (500,61) | | | |
|---|---|---|---|
| Ber.:<br>Gef.: | C 74,38<br>74,91 | H 6,04<br>6,33 | H 8,39<br>8,00 |

Massenspektrum: $M^+$ = 491.

Beispiel 13

(R,S)-2-Ethyl-8-methyl-5-[α-(2'-carboxybiphenyl-4-yl)-α-benzyloxy-methyl]-imidazo[1,2-a]pyridin

Hergestellt analog Beispiel 1c aus (R,S)-2-Ethyl-8-methyl-5-[α-(2'-tert.butyloxycarbonyl)biphenyl-4-yl)-α-benzyloxy-methyl]-imidazo[1,2-a]pyridin und Trifluoressigsäure.
Ausbeute: 17 % der Theorie,
Schmelzpunkt: 226 °C (Zers.)
$C_{31}H_{28}N_2O_3$ (476,58)
Massenspektrum: $M^+$ = 476.

Beispiel 14

2-n-Propyl-8-methyl-5-[α-(2'-carboxybiphenyl-4-yl)-α-ethoxycarbonylmethoxy-methyl]-imidazo[1,2-a]pyridin-semihydrat

Hergestellt analog Beispiel 1c aus 2-n-Propyl-8-methyl-5-[α-(2'-tert.butyloxycarbonyl)-α-ethoxycarbonylmethoxymethyl]-imidazo[1,2-a]pyridin und Trifluoressigsäure.
Ausbeute: 39 % der Theorie,
Schmelzpunkt: 115-120 °C (sintert ab 85 °C)

| $C_{29}H_{30}N_2O_5$ x 0,5 $H_2O$ (486,58) | | | |
|---|---|---|---|
| Ber.:<br>Gef.: | C 70,29<br>70,09 | H 6,31<br>6,52 | N 5,65<br>5,40 |

Massenspektrum: $M^+$ = 486.

Beispiel 15

2-Ethyl-8-methyl-5-[(2'-carboxybiphenyl-4-yl)methyl]imidazo[1,2-a]pyridin

Zu einer Mischung von 20 ml Trifluoressigsäure und 2,35 g (62 mMol) Natriumborhydrid wird bei 5 °C eine Lösung von 1,30 g (2,85 mMol) (R,S)-2-Ethyl-8-methyl-5-[α-[2'-tert.butyloxycarbonyl)biphenyl-4-yl)-α-hydroxy-methyl]imidazo[1,2-a]pyridin in 20 ml Methylenchlorid zugetropft. Die dabei gebildete Suspension wird 22 Stunden bei Raumtemperatur gerührt. Danach werden 1,0 g (2,6 mMol) Natriumborhydrid und 20 ml Methylenchlorid zugegeben und weitere 6 Stunden bei Raumtemperatur gerührt. Das Reaktionsgemisch wird mit Eiswasser zersetzt und mit Methylenchlorid extrahiert. Die organische Phase wird mit Wasser gewaschen, über Magnesiumsulfat getrocknet und eingedampft. Der Rückstand wird über eine Kieselgelsäule (Korngröße: 0,063 - 0,02 mm) gereinigt, wobei als Elutionsmittel Methylenchlorid/Methanol 9:1 und 3:1 verwendet wird. Die einheitlichen Fraktionen werden im Vakuum eingeengt.
Ausbeute: 0,1 g (9 % der Theorie),
Schmelzpunkt: 257-260 °C (Zers.)

| $C_{25}H_{24}N_2O_2$ (384,48) | | | |
|---|---|---|---|
| Ber. x 0,75 $H_2O$:<br>Gef. : | C 75,45<br>75,52 | H 6,46<br>6,56 | N 7,04<br>6,72 |

Massenspektrum: $M^+$ = 384

Beispiel 16

2-Ethyl-5-[4-(2-carboxyphenyl)phenoxy]imidazo[1,2-a]pyridin

a) 2-Ethyl-5-chlor-imidazo[1,2-a]pyridin

Hergestellt analog Beispiel 1a aus 2-Chlor-6-amino-pyridin und 1-Chlor-2-butanon.
Ausbeute: 44 % der Theorie,
Öl, $R_f$-Wert: 0,40 (Kieselgel; Laufmittel: Methylenchlorid/Xylol = 1:1)

b) 2-Ethyl-5-[4-(2-methoxycarbonyl)phenyl]phenoxy]imidazo[1,2-a]pyridin

1,0 g (5,5 mMol) 2-Ethyl-5-chlor-imidazo[1,2-a]pyridin, 685 mg Kalium-tert.butylat und 1,50 g (6,6 mMol) 2'-Methoxycarbonyl-4-hydroxy-biphenyl werden in 15 ml Dimethylsulfoxid gelöst und 24 Stunden bei Raumtemperatur gerührt. Anschließend wird das Reaktionsgemisch noch 2 Stunden auf 80 °C erhitzt. Nach Abkühlung wird auf Eiswasser gegossen und 2 x mit je 50 ml Essigester extrahiert. Die vereinigten organischen Phasen werden mit Wasser und gesättigter Natriumchloridlösung gewaschen, über Natriumsulfat getrocknet und eingedampft. Der Rückstand wird über eine Kieselgelsäule (Korngröße: 0,063 - 0,02 mm) gereinigt, wobei als Elutionsmittel Methylenchlorid/Ethanol 99:1 und 49:1 verwendet wird. Die einheitlichen Fraktionen werden im Vakuum eingeengt.
Ausbeute: 1,26 g (63 % der Theorie),
Öl, $R_f$-Wert: 0,35 (Kieselgel; Laufmittel: Methylethylketon/Xylol = 1:1)

c) 2-Ethyl-5-[4-(2-carboxyphenyl)phenoxy]imidazo[1,2-a]pyridin

1,25 g (3,3 mMol) 2-Ethyl-5-[4-(2-methoxycarbonyl)phenyl]-phenoxy]imidazo[1,2-a]pyridin werden in 25 ml Ethanol gelöst, mit 15 ml 2N Natronlauge versetzt und 1 Stunde unter Rückfluß erhitzt. Nach Abkühlung und Zugabe von 15 ml Wasser wird das Methanol im Vakuum abdestilliert und die wässrige Phase mit Essigester extrahiert. Die wässrige Phase wird mit Salzsäure auf pH 6 eingestellt. Der kristallisierte Niederschlag wird in der Kälte abgesaugt und getrocknet.
Ausbeute: 830 mg (69 % der Theorie),
Schmelzpunkt: 261-263 °C

| $C_{22}H_{18}N_2O_3$ (358,40) | | | |
|---|---|---|---|
| Ber.:<br>Gef.: | C 73,73<br>73.60 | H 5,06<br>5,09 | N 7,82<br>7,78 |

Beispiel 17

2-n-Propyl-8-methyl-5-[α-(2'-carboxybiphenyl-4-yl)-α-(2-carboxy-ethylcarbonyloxy)methyl]imidazo[1,2-a]-pyridin-dihydrat

a) 2-n-Propyl-8-methyl-5-[α-(2'-(tert.butoxycarbonyl)biphenyl-4-yl)-α-(2-carboxy-ethylcarbonyloxy)methyl]-imidazo[1,2-a]pyridin

Hergestellt analog Beispiel 4a aus 2-n-Propyl-8-methyl-5-[α-(2'-(tert.butoxycarbonyl)biphenyl-4-yl)-α-hydroxy-methyl]imidazo[1,2-a]pyridin und Bernsteinsäureanhydrid.
Ausbeute: 52 % der Theorie,
Schmelzpunkt: 101 °C (Zers.)

b) 2-n-Propyl-8-methyl-5-[α-(2'-carboxybiphenyl-4-yl)-α-(2-carboxy-ethylcarbonyloxy)methyl]imidazo[1,2-a]-pyridin-dihydrat

Hergestellt analog Beispiel 1c aus 2-n-Propyl-8-methyl-5-[α-(2'-(tert.butoxycarbonyl)biphenyl-4-yl)-α-(2-carboxy-ethylcarbonyloxy)methyl]imidazo[1,2-a]pyridin und Trifluoressigsäure.
Ausbeute: 78 % der Theorie,

Schmelzpunkt: ab 100 °C (Zers.)

| $C_{28}H_{28}N_2O_6$ 2 x $H_2O$ (536,59) | | | |
|---|---|---|---|
| Ber. x 2 $H_2O$:<br>Gef. : | C 64,91<br>65,29 | H 6,01<br>6,00 | N 5,22<br>5,21 |

Analog den Verfahren der vorliegenden Anmeldung können folgende Verbindungen erhalten werden:

(1) (R,S)-2-Ethyl-3,8-dimethyl-5-[α-(2'-carboxybiphenyl-4-yl)-α-hydroxy-methyl]imidazo[1,2-a]pyridin
(2) (R,S)-2-Ethyl-3-brom-8-methyl-5-[α-(2'-carboxybiphenyl-4-yl)-α-hydroxy-methyl]imidazo[1,2-a]pyridin
(3) (R,S)-2-Ethyl-7-methyl-5-[α-(2'-carboxybiphenyl-4-yl)-α-hydroxy-methyl]imidazo[1,2-a]pyridin
(4) (R,S)-2-Ethyl-6-methyl-5-[α-(2'-carboxybiphenyl-4-yl)-α-hydroxy-methyl]imidazo[1,2-a]pyridin
(5) (R,S)-2-Ethyl-6,8-dimethyl-5-[α-(2'-carboxybiphenyl-4-yl)-α-hydroxy-methyl]imidazo[1,2-a]pyridin
(6) (R,S)-2-Ethyl-7-trifluormethyl-5-[α-(2'-carboxybiphenyl-4-yl)-α-hydroxy-methyl]imidazo[1,2-a]pyridin
(7) (R,S)-2-Ethyl-6,8-dimethyl-5-[α-(2'-(1H-tetrazol-5-yl)biphenyl-4-yl)-α-hydroxy-methyl]imidazo[1,2-a]-pyridin
(8) (R,S)-2-Ethyl-8-methyl-5-[α-(2'-(1H-tetrazol-5-yl)biphenyl-4-yl)-α-hydroxy-methyl]imidazo[1,2-a]pyridin
(9) (R,S)-2-Ethyl-8-methyl-5-[α-(2'-carboxybiphenyl-4-yl)-α-ethoxy-methyl]imidazo[1,2-a]pyridin
(10) (R,S)-2-Ethyl-8-methyl-5-[α-(2'-carboxybiphenyl-4-yl)-α-n-propyloxy-methyl]imidazo[1,2-a]pyridin
(11) (R,S)-2-Ethyl-8-methyl-5-[α-(2'-carboxybiphenyl-4-yl)-α-isobutyloxy-methyl]imidazo[1,2-a]pyridin
(12) (R,S)-2-Ethyl-8-methyl-5-[α-(2'-(1H-tetrazol-5-yl)biphenyl-4-yl)-α-methoxy-methyl]imidazo[1,2-a]-pyridin
(13) (R,S)-2-Ethyl-8-methyl-5-[α-(2'-carboxybiphenyl-4-yl)-α-propionyloxy-methyl]imidazo[1,2-a]pyridin
(14) (R,S)-2-Ethyl-8-methyl-5-[α-(2'-carboxybiphenyl-4-yl)-α-butyryloxy-methyl]imidazo[1,2-a]pyridin
(15) (R,S)-2-Ethyl-8-methyl-5-[α-(2'-carboxybiphenyl-4-yl)-α-pivaloyloxy-methyl]imidazo[1,2-a]pyridin
(16) (R,S)-2-Ethyl-8-methyl-5-[α-(2'-carboxybiphenyl-4-yl)-α-benzoyloxy-methyl]imidazo[1,2-a]pyridin
(17) (R,S)-2-Ethyl-8-methyl-5-[α-(2'-carboxybiphenyl-4-yl)-α-phenylmethylcarbonyloxy-methyl]imidazo-[1,2-a]pyridin
(18) (R,S)-2-Ethyl-8-methyl-5-[α-(2'-(1H-tetrazol-5-yl)biphenyl-4-yl)-α-pivaloyloxy-methyl]imidazo[1,2-a]-pyridin
(19) (R,S)-2-Ethyl-8-methyl-5-[α-(2'-carboxybiphenyl-4-yl)-α-(n-butylamino-carbonyloxy)methyl]imidazo-[1,2-a]pyridin
(20) (R,S)-2-Ethyl-8-methyl-5-[α-(2'-carboxybiphenyl-4-yl)-α-(phenylamino-carbonyloxy)methyl]imidazo-[1,2-a]pyridin
(21) (R,S)-2-Ethyl-8-methyl-5-[α-(2'-carboxybiphenyl-4-yl)-α-(benzylamino-carbonyloxy)methyl]imidazo-[1,2-a]pyridin
(22) 2-Ethyl-8-methyl-5-[(2'-(1H-tetrazol-5-yl)biphenyl-4-yl)methyl]imidazo[1,2-a]pyridin
(23) (R,S)-2-Ethyl-8-methyl-5-[α-(2'-(1H-tetrazol-5-yl)biphenyl-4-yl)-α-(cyclohexylaminocarbonyloxy)-methyl]imidazo[1,2-a]pyridin
(24) (R,S)-2-n-Propyl-3,8-dimethyl-5-[α-(2'-carboxybiphenyl-4-yl)-α-hydroxy-methyl]imidazo[1,2-a]pyridin
(25) (R,S)-2-n-Propyl-3-brom-8-methyl-5-[α-(2'-carboxybiphenyl-4-yl)-α-hydroxy-methyl]imidazo[1,2-a]-pyridin
(26) (R,S)-2-n-Propyl-7-methyl-5-[α-(2'-carboxybiphenyl-4-yl)-α-hydroxy-methyl]imidazo[1,2-a]pyridin
(27) (R,S)-2-n-Propyl-6-methyl-5-[α-(2'-carboxybiphenyl-4-yl)-α-hydroxy-methyl]imidazo[1,2-a]pyridin
(28) (R,S)-2-n-Propyl-6,8-dimethyl-5-[a-(2'-carboxybiphenyl-4-yl)-α-hydroxy-methyl]imidazo[1,2-a]pyridin
(29) (R,S)-2-n-Propyl-7-trifluormethyl-5-[α-(2'-carboxybiphenyl-4-yl)-α-hydroxy-methyl]imidazo[1,2-a]-pyridin
(30) (R,S)-2-n-Propyl-6,8-dimethyl-5-[α-(2'-(1H-tetrazol-5-yl)biphenyl-4-yl)-α-hydroxy-methyl]imidazo[1,2-a]pyridin
(31) (R,S)-2-n-Propyl-8-methyl-5-[α-(2'-(1H-tetrazol-5-yl)biphenyl-4-yl)-α-hydroxy-methyl]imidazo[1,2-a]-pyridin
(32) (R,S)-2-n-Propyl-8-methyl-5-[α-(2'-carboxybiphenyl-4-yl)-α-ethoxy-methyl]imidazo[1,2-a]pyridin
(33) (R,S)-2-n-Propyl-8-methyl-5-[α-(2'-carboxybiphenyl-4-yl)-α-n-propyloxy-methyl]imidazo[1,2-a]pyridin
(34) (R,S)-2-n-Propyl-8-methyl-5-[α-(2'-carboxybiphenyl-4-yl)-α-isobutyloxy-methyl]imidazo[1,2-a]pyridin
(35) (R,S)-2-n-Propyl-8-methyl-5-[α-(2'-(1H-tetrazol-5-yl)-biphenyl-4-yl)-α-methoxy-methyl]imidazo[1,2-a]-pyridin
(36) (R,S)-2-n-Propyl-8-methyl-5-[a-(2'-carboxybiphenyl-4-yl)-α-propionyloxy-methyl]imidazo[1,2-a]pyridin
(37) (R,S)-2-n-Propyl-8-methyl-5-[α-(2'-carboxybiphenyl-4-yl)-α-butyryloxy-methyl]imidazo[1,2-a]pyridin

(38) (R,S)-2-n-Propyl-8-methyl-5-[α-(2'-carboxybiphenyl-4-yl)-α-pivaloyloxy-methyl]imidazo[1,2-a]pyridin
(39) (R,S)-2-n-Propyl-8-methyl-5-[α-(2'-carboxybiphenyl-4-yl)-α-cyclohexylcarbonyloxy-methyl]imidazo-[1,2-a]pyridin
(40) (R,S)-2-n-Propyl-8-methyl-5-[α-(2'-carboxybiphenyl-4-yl)-α-phenylmethylcarbonyloxy-methyl]imidazo-[1,2-a]pyridin
(41) (R,S)-2-n-Propyl-8-methyl-5-[α-(2'-(1H-tetrazol-5-yl)-biphenyl-4-yl)-α-pivaloyloxy-methyl]imidazo[1,2-a]pyridin
(42) (R,S)-2-n-Propyl-8-methyl-5-[α-(2'-carboxybiphenyl-4-yl)-α-(n-butylamino-carbonyloxy)methyl]-imidazo[1,2-a]-pyridin
(43) (R,S)-2-n-Propyl-8-methyl-5-[α-(2'-carboxybiphenyl-4-yl)-α-(phenylamino-carbonyloxy)methyl]-imidazo[1,2-a]-pyridin
(44) (R,S)-2-n-Propyl-8-methyl-5-[α-(2'-carboxybiphenyl-4-yl)-α-(cyclohexylamino-carbonyloxy)methyl]-imidazo[1,2-a]-pyridin
(45) (R,S)-2-n-Propyl-8-methyl-5-[α-(2'-carboxybiphenyl-4-yl)-α-(benzylamino-carbonyloxy)methyl]-imidazo[1,2-a]-pyridin
(46) 2-n-Propyl-8-methyl-5-[(2'-(1H-tetrazol-5-yl)biphenyl-4-yl)methyl]imidazo[1,2-a]pyridin
(47) (R,S)-2-n-Propyl-8-methyl-5-[α-(2'-(1H-tetrazol-5-yl)-biphenyl-4-yl)-α-(cyclohexylaminocarbonyloxy)-methyl]imidazo[1,2-a]pyridin
(48) (R,S)-2-Cyclopropyl-3,8-dimethyl-5-[α-(2'-carboxybiphenyl-4-yl)-α-hydroxy-methyl]imidazo[1,2-a]-pyridin
(49) (R,S)-2-Cyclopropyl-3-brom-8-methyl-5-[α-(2'-carboxybiphenyl-4-yl)-α-hydroxy-methyl]imidazo[1,2-a]pyridin
(50) (R,S)-2-Cyclopropyl-7-methyl-5-[α-(2'-carboxybiphenyl-4-yl)-α-hydroxy-methyl]imidazo[1,2-a]pyridin
(51) (R,S)-2-Cyclopropyl-6-methyl-5-[α-(2'-carboxybiphenyl-4-yl)-α-hydroxy-methyl]imidazo[1,2-a]pyridin
(52) (R,S)-2-Cyclopropyl-6,8-dimethyl-5-[α-(2'-carboxybiphenyl-4-yl)-α-hydroxy-methyl]imidazo[1,2-a]-pyridin
(53) (R,S)-2-Cyclopropyl-7-trifluormethyl-5-[α-(2'-carboxybiphenyl-4-yl)-α-hydroxy-methyl]imidazo[1,2-a]-pyridin
(54) (R,S)-2-Cyclopropyl-6,8-dimethyl-5-[α-(2'-(1H-tetrazol-5-yl)biphenyl-4-yl)-α-hydroxy-methyl]imidazo-[1,2-a]-pyridin
(55) (R,S)-2-Cyclopropyl-8-methyl-5-[α-(2'-(1H-tetrazol-5-yl)biphenyl-4-yl)-α-hydroxy-methyl]imidazo[1,2-a]pyridin
(56) (R,S)-2-Cyclopropyl-8-methyl-5-[α-(2'-carboxybiphenyl-4-yl)-α-ethoxy-methyl]imidazo[1,2-a]pyridin
(57) (R,S)-2-Cyclopropyl-8-methyl-5-[α-(2'-carboxybiphenyl-4-yl)-α-n-propyloxy-methyl]imidazo[1,2-a]-pyridin
(58) (R,S)-2-Cyclopropyl-8-methyl-5-[α-(2'-carboxybiphenyl-4-yl)-α-isobutyloxy-methyl]imidazo[1,2-a]-pyridin
(59) (R,S)-2-Cyclopropyl-8-methyl-5-[α-(2'-(1H-tetrazol-5-yl)biphenyl-4-yl)-α-methoxy-methyl]imidazo[1,2-a]pyridin
(60) (R,S)-2-Cyclopropyl-8-methyl-5-[α-(2'-carboxybiphenyl-4-yl)-α-propionyloxy-methyl]imidazo[1,2-a]-pyridin
(61) (R,S)-2-Cyclopropyl-8-methyl-5-[α-(2'-carboxybiphenyl-4-yl)-α-butyryloxy-methyl]imidazo[1,2-a]-pyridin
(62) (R,S)-2-Cyclopropyl-8-methyl-5-[α-(2'-carboxybiphenyl-4-yl)-α-pivaloyloxy-methyl]imidazo[1,2-a]-pyridin
(63) (R,S)-2-Cyclopropyl-8-methyl-5-[α-(2'-carboxybiphenyl-4-yl)-α-cyclohexylcarbonyloxy-methyl]-imidazo[1,2-a]-pyridin
(64) (R,S)-2-Cyclopropyl-8-methyl-5-[α-(2'-carboxybiphenyl-4-yl)-α-benzoyloxy-methyl]imidazo[1,2-a]-pyridin
(65) (R,S)-2-Cyclopropyl-8-methyl-5-[α-(2'-carboxybiphenyl-4-yl)-α-phenylmethylcarbonyloxy-methyl]-imidazo[1,2-a]-pyridin
(66) (R,S)-2-Cyclopropyl-8-methyl-5-[α-(2'-(1H-tetrazol-5-yl)biphenyl-4-yl)-α-pivaloyloxy-methyl]imidazo-[1,2-a]-pyridin
(67) (R,S)-2-Cyclopropyl-8-methyl-5-[α-(2'-carboxybiphenyl-4-yl)-α-(n-butylamino-carbonyloxy)methyl]-imidazo[1,2-a]pyridin
(68) (R,S)-2-Cyclopropyl-8-methyl-5-[α-(2'-carboxybiphenyl-4-yl)-α-(phenylamino-carbonyloxy)methyl]-imidazo[1,2-a]pyridin

(69) (R,S)-2-Cyclopropyl-8-methyl-5-[α-(2'-carboxybiphenyl-4-yl)-α-(cyclohexylamino-carbonyloxy)-methyl]imidazo[1,2-a]pyridin
(70) (R,S)-2-Cyclopropyl-8-methyl-5-[α-(2'-carboxybiphenyl-4-yl)-α-(benzylamino-carbonyloxy)methyl]-imidazo[1,2-a]-pyridin
(71) 2-Cyclopropyl-8-methyl-5-[(2'-(1H-tetrazol-5-yl)-biphenyl-4-yl)methyl]imidazo[1,2-a]pyridin
(72) (R,S)-2-Cyclopropyl-8-methyl-5-[α-(2'-(1H-tetrazol-5-yl)biphenyl-4-yl)-α-(cyclohexylaminocarbonyloxy)methyl]-imidazo[1,2-a]pyridin
(73) (R,S)-2-n-Butyl-3,8-dimethyl-5-[α-(2'-carboxybiphenyl-4-yl)-α-hydroxy-methyl]imidazo[1,2-a]pyridin
(74) (R,S)-2-n-Butyl-3-brom-8-methyl-5-[α-(2'-carboxybiphenyl-4-yl)-α-hydroxy-methyl]imidazo[1,2-a]-pyridin
(75) (R,S)-2-n-Butyl-7-methyl-5-[α-(2'-carboxybiphenyl-4-yl)-α-hydroxy-methyl]imidazo[1,2-a]pyridin
(76) (R,S)-2-n-Butyl-6-methyl-5-[α-(2'-carboxybiphenyl-4-yl)-α-hydroxy-methyl]imidazo[1,2-a]pyridin
(77) (R,S)-2-n-Butyl-6,8-dimethyl-5-[α-(2'-carboxybiphenyl-4-yl)-α-hydroxy-methyl]imidazo[1,2-a]pyridin
(78) (R,S)-2-n-Butyl-7-trifluormethyl-5-[α-(2'-carboxybiphenyl-4-yl)-α-hydroxy-methyl]imidazo[1,2-a]pyridin
(79) (R,S)-2-n-Butyl-6,8-dimethyl-5-[α-(2'-(1H-tetrazol-5-yl)biphenyl-4-yl)-α-hydroxy-methyl]imidazo[1,2-a]-pyridin
(80) (R,S)-2-n-Butyl-8-methyl-5-[α-(2'-(1H-tetrazol-5-yl)-biphenyl-4-yl)-α-hydroxy-methyl]imidazo[1,2-a]-pyridin
(81) (R,S)-2-n-Butyl-8-methyl-5-[α-(2'-carboxybiphenyl-4-yl)-α-ethoxy-methyl]imidazo[1,2-a]pyridin
(82) (R,S)-2-n-Butyl-8-methyl-5-[α-(2'-carboxybiphenyl-4-yl)-α-n-propyloxy-methyl]imidazo[1,2-a]pyridin
(83) (R,S)-2-n-Butyl-8-methyl-5-[α-(2'-carboxybiphenyl-4-yl)-α-isobutyloxy-methyl]imidazo[1,2-a]pyridin
(84) (R,S)-2-n-Butyl-8-methyl-5-[α-(2'-(1H-tetrazol-5-yl)biphenyl-4-yl)-α-methoxy-methyl]imidazo[1,2-a]-pyridin
(85) (R,S)-2-n-Butyl-8-methyl-5-[α-(2'-carboxybiphenyl-4-yl)-α-propionyloxy-methyl]imidazo[1,2-a]pyridin
(86) (R,S)-2-n-Butyl-8-methyl-5-[α-(2'-carboxybiphenyl-4-yl)-α-butyryloxy-methyl]imidazo[1,2-a]pyridin
(87) (R,S)-2-n-Butyl-8-methyl-5-[α-(2'-carboxybiphenyl-4-yl)-α-pivaloyloxy-methyl]imidazo[1,2-a]pyridin
(88) (R,S)-2-n-Butyl-8-methyl-5-[α-(2'-carboxybiphenyl-4-yl)-α-cyclohexylcarbonyloxy-methyl]imidazo[1,2-a]pyridin
(89) (R,S)-2-n-Butyl-8-methyl-5-[α-(2'-carboxybiphenyl-4-yl)-α-benzoyloxy-methyl]imidazo[1,2-a]pyridin
(90) (R,S)-2-n-Butyl-8-methyl-5-[α-(2'-carboxybiphenyl-4-yl)-α-phenylmethylcarbonyloxy-methyl]imidazo-[1,2-a]-pyridin
(91) (R,S)-2-n-Butyl-8-methyl-5-[α-(2'-(1H-tetrazol-5-yl)-biphenyl-4-yl)-α-pivaloyloxy-methyl]imidazo[1,2-a]-pyridin
(92) (R,S)-2-n-Butyl-8-methyl-5-[α-(2'-carboxybiphenyl-4-yl)-α-(n-butylamino-carbonyloxy)methyl]imidazo-[1,2-a]-pyridin
(93) (R,S)-2-n-Butyl-8-methyl-5-[α-(2'-carboxybiphenyl-4-yl)-α-(phenylamino-carbonyloxy)methyl]imidazo-[1,2-a]pyridin
(94) (R,S)-2-n-Butyl-8-methyl-5-[α-(2'-carboxybiphenyl-4-yl)-α-(cyclohexylamino-carbonyloxy)methyl]-imidazo[1,2-a]-pyridin
(95) (R,S)-2-n-Butyl-8-methyl-5-[α-(2'-carboxybiphenyl-4-yl)-α-(benzylamino-carbonyloxy)methyl]imidazo-[1,2-a]pyridin
(96) 2-n-Butyl-8-methyl-5-[(2'-(1H-tetrazol-5-yl)biphenyl-4-yl)methyl]imidazo[1,2-a]pyridin
(97) (R,S)-2-n-Butyl-8-methyl-5-[α-(2'-(1H-tetrazol-5-yl)-biphenyl-4-yl)-α-(cyclohexylaminocarbonyloxy)-methyl]imidazo[1,2-a]pyridin
(98) (R,S)-2-Methoxy-3,8-dimethyl-5-[α-(2'-carboxybiphenyl-4-yl)-α-hydroxy-methyl]imidazo[1,2-a]pyridin
(99) (R,S)-2-Methoxy-3-brom-8-methyl-5-[α-(2'-carboxybiphenyl-4-yl)-α-hydroxy-methyl]imidazo[1,2-a]-pyridin
(100) (R,S)-2-Methoxy-7-methyl-5-[α-(2'-carboxybiphenyl-4-yl)-α-hydroxy-methyl]imidazo[1,2-a]pyridin
(101) (R,S)-2-Methoxy-6-methyl-5-[α-(2'-carboxybiphenyl-4-yl)-α-hydroxy-methyl]imidazo[1,2-a]pyridin
(102) (R,S)-2-Methoxy-6,8-dimethyl-5-[α-(2'-carboxybiphenyl-4-yl)-α-hydroxy-methyl]imidazo[1,2-a]pyridin
(103) (R,S)-2-Methoxy-7-trifluormethyl-5-[α-(2'-carboxybiphenyl-4-yl)-α-hydroxy-methyl]imidazo[1,2-a]-pyridin
(104) (R,S)-2-Methoxy-6,8-dimethyl-5-[α-(2'-(1H-tetrazol-5-yl)biphenyl-4-yl)-α-hydroxy-methyl]imidazo[1,2-a]pyridin
(105) (R,S)-2-Methoxy-8-methyl-5-[α-(2'-(1H-tetrazol-5-yl)-biphenyl-4-yl)-α-hydroxy-methyl]imidazo[1,2-a]-pyridin
(106) (R,S)-2-Methoxy-8-methyl-5-[α-(2'-carboxybiphenyl-4-yl)-α-ethoxy-methyl]imidazo[1,2-a]pyridin
(107) (R,S)-2-Methoxy-8-methyl-5-[α-(2'-carboxybiphenyl-4-yl)-α-n-propyloxy-methyl]imidazo[1,2-a]pyridin

(108) (R,S)-2-Methoxy-8-methyl-5-[α-(2'-carboxybiphenyl-4-yl)-α-isobutyloxy-methyl]imidazo[1,2-a]pyridin
(109) (R,S)-2-Methoxy-8-methyl-5-[α-(2'-(1H-tetrazol-5-yl)-biphenyl-4-yl)-α-methoxy-methyl]imidazo[1,2-a]pyridin
(110) (R,S)-2-Methoxy-8-methyl-5-[α-(2'-carboxybiphenyl-4-yl)-α-propionyloxy-methyl]imidazo[1,2-a]-pyridin
(111) (R,S)-2-Methoxy-8-methyl-5-[α-(2'-carboxybiphenyl-4-yl)-α-butyryloxy-methyl]imidazo[1,2-a]pyridin
(112) (R,S)-2-Methoxy-8-methyl-5-[α-(2'-carboxybiphenyl-4-yl)-α-pivaloyloxy-methyl]imidazo[1,2-a]pyridin
(113) (R,S)-2-Methoxy-8-methyl-5-[α-(2'-carboxybiphenyl-4-yl)-α-cyclohexylcarbonyloxy-methyl]imidazo-[1,2-a]pyridin
(114) (R,S)-2-Methoxy-8-methyl-5-[α-(2'-carboxybiphenyl-4-yl)-α-benzoyloxy-methyl]imidazo[1,2-a]pyridin
(115) (R,S)-2-Methoxy-8-methyl-5-[α-(2'-carboxybiphenyl-4-yl)-α-phenylmethylcarbonyloxy-methyl]-imidazo[1,2-a]pyridin
(116) (R,S)-2-Methoxy-8-methyl-5-[α-(2'-(1H-tetrazol-5-yl)-biphenyl-4-yl)-α-pivaloyloxy-methyl]imidazo-[1,2-a]pyridin
(117) (R,S)-2-Methoxy-8-methyl-5-[α-(2'-carboxybiphenyl-4-yl)-α-(n-butylamino-carbonyloxy)methyl]-imidazo[1,2-a]-pyridin
(118) (R,S)-2-Methoxy-8-methyl-5-[α-(2'-carboxybiphenyl-4-yl)-α-(phenylamino-carbonyloxy)methyl]-imidazo[1,2-a]-pyridin
(119) (R,S)-2-Methoxy-8-methyl-5-[α-(2'-carboxybiphenyl-4-yl)-α-(cyclohexylamino-carbonyloxy)methyl]-imidazo[1,2-a]-pyridin
(120) (R,S)-2-Methoxy-8-methyl-5-[α-(2'-carboxybiphenyl-4-yl)-α-(benzylamino-carbonyloxy)methyl]-imidazo[1,2-a]-pyridin
(121) 2-Methoxy-8-methyl-5-[(2'-(1H-tetrazol-5-yl)biphenyl-4-yl)methyl]imidazo[1,2-a]pyridin
(122) (R,S)-2-Methoxy-8-methyl-5-[α-(2'-(1H-tetrazol-5-yl)-biphenyl-4-yl)-α-(cyclohexylaminocarbonyloxy)-methyl]imidazo[1,2-a]pyridin
(123) (R,S)-2-Ethoxy-3,8-dimethyl-5-[α-(2'-carboxybiphenyl-4-yl)-α-hydroxy-methyl]imidazo[1,2-a]pyridin
(124) (R,S)-2-Ethoxy-3-brom-8-methyl-5-[α-(2'-carboxybiphenyl-4-yl)-α-hydroxy-methyl]imidazo[1,2-a]-pyridin
(125) (R,S)-2-Ethoxy-7-methyl-5-[α-(2'-carboxybiphenyl-4-yl)-α-hydroxy-methyl]imidazo[1,2-a]pyridin
(126) (R,S)-2-Ethoxy-6-methyl-5-[α-(2'-carboxybiphenyl-4-yl)-α-hydroxy-methyl]imidazo[1,2-a]pyridin
(127) (R,S)-2-Ethoxy-6,8-dimethyl-5-[α-(2'-carboxybiphenyl-4-yl)-α-hydroxy-methyl]imidazo[1,2-a]pyridin
(128) (R,S)-2-Ethoxy-7-trifluormethyl-5-[α-(2'-carboxybiphenyl-4-yl)-α-hydroxy-methyl]imidazo[1,2-a]-pyridin
(129) (R,S)-2-Ethoxy-6,8-dimethyl-5-[α-(2'-(1H-tetrazol-5-yl)biphenyl-4-yl)-α-hydroxy-methyl]imidazo[1,2-a]pyridin
(130) (R,S)-2-Ethoxy-8-methyl-5-[α-(2'-(1H-tetrazol-5-yl)-biphenyl-4-yl)-α-hydroxy-methyl]imidazo[1,2-a]-pyridin
(131) (R,S)-2-Ethoxy-8-methyl-5-[α-(2'-carboxybiphenyl-4-yl)-α-ethoxy-methyl]imidazo[1,2-a]pyridin
(132) (R,S)-2-Ethoxy-8-methyl-5-[α-(2'-carboxybiphenyl-4-yl)-α-n-propyloxy-methyl]imidazo[1,2-a]pyridin
(133) (R,S)-2-Ethoxy-8-methyl-5-[α-(2'-carboxybiphenyl-4-yl)-α-isobutyloxy-methyl]imidazo[1,2-a]pyridin
(134) (R,S)-2-Ethoxy-8-methyl-5-[α-(2'-(1H-tetrazol-5-yl)-biphenyl-4-yl)-α-methoxy-methyl]imidazo[1,2-a]-pyridin
(135) (R,S)-2-Ethoxy-8-methyl-5-[α-(2'-carboxybiphenyl-4-yl)-α-propionyloxy-methyl]imidazo[1,2-a]pyridin
(136) (R,S)-2-Ethoxy-8-methyl-5-[α-(2'-carboxybiphenyl-4-yl)-α-butyryloxy-methyl]imidazo[1,2-a]pyridin
(137) (R,S)-2-Ethoxy-8-methyl-5-[α-(2'-carboxybiphenyl-4-yl)-α-pivaloyloxy-methyl]imidazo[1,2-a]pyridin
(138) (R,S)-2-Ethoxy-8-methyl-5-[α-(2'-carboxybiphenyl-4-yl)-α-cyclohexylcarbonyloxy-methyl]imidazo-[1,2-a]pyridin
(139) (R,S)-2-Ethoxy-8-methyl-5-[α-(2'-carboxybiphenyl-4-yl)-α-benzoyloxy-methyl]imidazo[1,2-a]pyridin
(140) (R,S)-2-Ethoxy-8-methyl-5-[α-(2'-carboxybiphenyl-4-yl)-α-phenylmethylcarbonyloxy-methyl]imidazo-[1,2-a]-pyridin
(141) (R,S)-2-Ethoxy-8-methyl-5-[α-(2'-(1H-tetrazol-5-yl)-biphenyl-4-yl)-α-pivaloyloxy-methyl]imidazo[1,2-a]pyridin
(142) (R,S)-2-Ethoxy-8-methyl-5-[α-(2'-carboxybiphenyl-4-yl)-α-(n-butylamino-carbonyloxy)methyl]-imidazo[1,2-a]-pyridin
(143) (R,S)-2-Ethoxy-8-methyl-5-[α-(2'-carboxybiphenyl-4-yl)-α-(phenylamino-carbonyloxy)methyl]-imidazo[1,2-a]-pyridin
(144) (R,S)-2-Ethoxy-8-methyl-5-[α-(2'-carboxybiphenyl-4-yl)-α-(cyclohexylamino-carbonyloxy)methyl]-imidazo[1,2-a]-pyridin

(145) (R,S)-2-Ethoxy-8-methyl-5-[α-(2'-carboxybiphenyl-4-yl)-α-(benzylamino-carbonyloxy)methyl]-imidazo[1,2-a]-pyridin

(146) 2-Ethoxy-8-methyl-5-[(2'-(1H-tetrazol-5-yl)biphenyl-4-yl)methyl]imidazo[1,2-a]pyridin

(147) (R,S)-2-Ethoxy-8-methyl-5-[α-(2'-(1H-tetrazol-5-yl)-biphenyl-4-yl)-α-(cyclohexylaminocarbonyloxy)-methyl]imidazo[1,2-a]pyridin

(148) (R,S)-2-Ethyl-3-carboxy-8-methyl-5-[α-(2'-carboxybiphenyl-4-yl)-α-hydroxy-methyl]imidazo[1,2-a]-pyridin

(149) (R,S)-2-Ethyl-3-ethoxycarbonyl-8-methyl-5-[α-(2'-carboxybiphenyl-4-yl)-α-hydroxy-methyl]imidazo-[1,2-a]pyridin

(150) (R,S)-2-n-Propyl-3-carboxy-8-methyl-5-[α-(2'-carboxybiphenyl-4-yl)-α-hydroxy-methyl]imidazo[1,2-a]pyridin

(151) (R,S)-2-n-Propyl-3-ethoxycarbonyl-8-methyl-5-[α-(2'-carboxybiphenyl-4-yl)-α-hydroxy-methyl]-imidazo[1,2-a]pyridin

(152) (R,S)-2-Cyclopropyl-3-carboxy-8-methyl-5-[α-(2'-carboxybiphenyl-4-yl)-α-hydroxy-methyl]imidazo-[1,2-a]pyridin

(153) (R,S)-2-Cyclopropyl-3-ethoxycarbonyl-8-methyl-5-[α-(2'-carboxybiphenyl-4-yl)-α-hydroxy-methyl]-imidazo[1,2-a]-pyridin

(154) (R,S)-2-n-Butyl-3-carboxy-8-methyl-5-[α-(2'-carboxy--biphenyl-4-yl)-α-hydroxy-methyl]imidazo[1,2-a]pyridin

(155) (R,S)-2-n-Butyl-3-ethoxycarbonyl-8-methyl-5-[α-(2'-carboxybiphenyl-4-yl)-α-hydroxy-methyl]-imidazo[1,2-a]pyridin

(156) (R,S)-2-Methoxy-3-carboxy-8-methyl-5-[α-(2'-carboxybiphenyl-4-yl)-α-hydroxy-methyl]imidazo[1,2-a]pyridin

(157) (R,S)-2-Methoxy-3-ethoxycarbonyl-8-methyl-5-[α-(2'-carboxybiphenyl-4-yl)-α-hydroxy-methyl]-imidazo[1,2-a]pyridin

(158) (R,S)-2-Ethoxy-3-carboxy-8-methyl-5-[α-(2'-carboxybiphenyl-4-yl)-α-hydroxy-methyl]imidazo[1,2-a]-pyridin

(159) (R,S)-2-Ethoxy-3-ethoxycarbonyl-8-methyl-5-[α-(2'-carboxybiphenyl-4-yl)-α-hydroxy-methyl]imidazo-[1,2-a]pyridin

Bei den nachfolgenden pharmazeutischen Anwendungsbeispielen kann als Wirksubstanz jede geeignete Verbindung der Formel I, insbesondere diejenigen in denen $R_e$ eine Carboxy- oder 1H-Tetrazolylgruppe darstellt, eingesetzt werden:

Beispiel I

Ampullen, enthaltend 50 mg Wirkstoff pro 5 ml

| | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

Ampullen, enthaltend 100 mg Wirkstoff pro 5 ml

| | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

Tabletten, enthaltend 50 mg Wirkstoff

| | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1.5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50 °C getrocknet und erneut gesiebt.

Nach Zumischen des Schmiermittels wird das Granulat auf einer Tablettiermaschine verpresst.

Beispiel IV

Dragees, enthaltend 50 mg Wirkstoff

| | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1.0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

Dragees, enthaltend 100 mg Wirkstoff

| | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1.2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45 ° C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

Kapseln, enthaltend 250 mg Wirkstoff

| | |
|---|---|
| Wirkstoff | 250,0 mg |
| Maisstärke | 68,5 mg |
| Magnesiumstearat | 1.5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

Orale Suspension, enthaltend 50 mg Wirkstoff pro 5 ml

| | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70 ° C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

Suppositorien, enthaltend 100 mg Wirkstoff

| | |
|---|---|
| Wirkstoff | 100,0 mg |
| Adeps solidus | 1600.0 mg |
| | 1700,0 mg |

Herstellung:

Das Hartfett wird geschmolzen. Bei 40 ° C wird die gemahlene Wirksubstanz in der Schmelze homogen dispergiert. Es wird auf 38 ° C abgekühlt und in schwach vorgekühlte Suppositorienformen ausgegossen.

**Patentansprüche**

**1.** Imidazo[1,2-a]pyridine der allgemeinen Formel

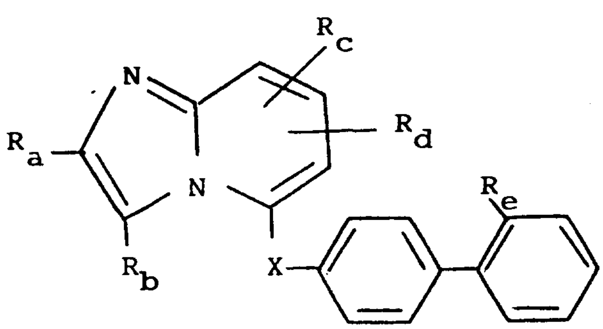

, (I)

in der
$R_a$ eine geradkettige oder verzweigte Alkylgruppe mit 1 bis 6 Kohlenstoffatomen, eine Cycloalkylgruppe, eine Alkylgruppe, die durch eine Alkoxygruppe substituiert ist, oder eine Alkoxygruppe mit 1 bis 4

Kohlenstoffatomen,
$R_b$ ein Wasserstoff-, Fluor-, Chlor- oder Bromatom, eine Alkyl-, Hydroxymethyl-, Trifluormethyl-, Formyl-, Carboxy-, Alkoxycarbonyl-, Cyano-, Nitro-, $NH_2CH_2$-, $R_1NHCH_2$- oder $R_1NR_2CH_2$-Gruppe, wobei
$R_1$ und $R_2$, die gleich oder verschieden sein können, Alkylgruppen mit 1 bis 6 Kohlenstoffatomen, Cycloalkyl-, Cycloalkylalkyl-, Phenyl- oder Phenylalkylgruppen oder
$R_1$ und $R_2$ zusammen eine n-Alkylengruppe mit 4 bis 6 Kohlenstoffatomen darstellen,
$R_c$ ein Wasserstoff-, Fluor-, Chlor- oder Bromatom, eine gegebenenfalls durch eine Alkoxy- oder Phenylalkoxygruppe substituierte Alkylgruppe, eine Alkoxy-, Phenylalkoxy-, Trifluormethyl-, $H_2N$-, $R_1NH$- oder $R_1NR_2$-Gruppe, wobei $R_1$ und $R_2$ wie vorstehend erwähnt definiert sind,
$R_d$ ein Wasserstoffatom oder eine Alkylgruppe,
$R_e$ eine Carboxygruppe, eine in-vivo in eine Carboxygruppe überführbare Gruppe, eine Cyano-, 1H-Tetrazolyl-, 1-Triphenylmethyl-tetrazolyl-, Alkansulfonylaminocarbonyl-, Phenylsulfonylaminocarbonyl-, Phenylalkansulfonylaminocarbonyl-, Trifluormethansulfonylaminocarbonyl-, Phosphino-, O-Alkyl-phosphino-, O-Aralkyl-phosphino-, Phosphono-, O-Alkyl-phosphono-, O-Aralkyl-phosphono-, O,O-Dialkylphosphono-, Phosphono-methyl-, O-Alkyl-phosphono-methyl-, O-Aralkyl-phosphono-methyl-, O-Aryl-phosphono-methyl-, O,O-Dialkyl-phosphono-methyl-, Phosphato-, O-Alkyl-phosphato-, O-Aralkyl-phosphato-, O-Aryl-phosphato- oder O,O-Dialkyl-phosphorylgruppe,
X ein Sauerstoffatom, eine gegebenenfalls durch eine Formyl-, $R_1$- oder $R_1CO$-Gruppe substituierte Iminogruppe, eine -CO-, -(HON=C)- oder -($R_3CR_4$)- Gruppe, wobei
$R_3$ ein Wasserstoffatom oder eine Alkylgruppe und
$R_4$ ein Wasserstoffatom, eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe, wobei die Heteroarylgruppe über eine Kohlenstoff-Kohlenstoff-Bindung mit der Alkoxygruppe verknüpft ist,
eine in 2-, 3- oder 4-Stellung durch eine Heteroarylgruppe substituierte Alkoxygruppe, wobei die Heteroarylgruppe über eine Kohlenstoff-Stickstoff-Bindung mit der Alkoxygruppe verknüpft ist,
eine Hydroxy-, Dialkylphosphonomethoxy-, Azido-, CHO-O-, $R_1O$-, $R_5NR_6$-, $R_1CO$-O-, $R_1O$-CO-O-, CHO-$NR_5$-, $R_1$-CO-$NR_7$-, $R_1O$-CO-$NR_5$-, $R_5NR_6$-CO-O-, $R_1SO_2$-O-, $R_5NR_6$-CO-$NR_5$- oder $R_1SO_2$-$NR_7$-Gruppe oder
$R_3$ und $R_4$ zusammen eine 1,2-Ethylendioxy- oder 1,3-n-Propylendioxygruppe darstellen, wobei in den vorstehend erwähnten Gruppen $R_1$ wie vorstehend erwähnt definiert ist,
$R_5$ und $R_6$, die gleich oder verschieden sein können, Wasserstoffatome oder die für $R_1$ und $R_2$ eingangs erwähnten Bedeutungen besitzen,
$R_7$ ein Wasserstoffatom oder eine Alkylgruppe oder $R_1$ und $R_7$ zusammen eine n-Alkylengruppe mit 3 bis 5 Kohlenstoffatomen darstellen, bedeuten,
wobei, soweit nichts anderes erwähnt wurde, ein vorstehend erwähnter Alkyl- oder Alkoxyteil jeweils 1 bis 4 Kohlenstoffatome sowie ein vorstehend erwähnter Cycloalkylteil jeweils 3 bis 7 Kohlenstoffatome enthalten kann, und
unter "eine Arylgruppe" eine gegebenenfalls durch ein Fluor-, Chlor- oder Bromatom, durch eine Hydroxy-, Alkyl-, Alkoxy-, Phenylalkoxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Trifluormethyl-, Alkanoyl-, Aminosulfonyl-, Alkylaminosulfonyl- oder Dialkylaminosulfonylgruppe mono- oder disubstituierte Phenylgruppe, wobei der Alkylteil jeweils 1 bis 4 Kohlenstoffatome enthalten kann, oder eine Naphthylgruppe und
unter einer "Heteroarylgruppe" ein über ein Kohlenstoffatom oder über eine Iminogruppe gebundener 5-gliedriger heteroaromatischer Ring, der eine Iminogruppe, ein Sauerstoff- oder Schwefelatom oder eine Iminogruppe und ein Sauerstoff-, Schwefel- oder Stickstoffatom enthält, oder ein über ein Kohlenstoffatom gebundener 6-gliedriger heteroaromatischer Ring, der 1 oder 2 Stickstoffatome enthält, wobei die vorstehend erwähnten heteroaromatischen Ringe im Kohlenstoffgerüst durch eine Alkylgruppe mit 1 bis 6 Kohlenstoffatomen oder durch eine Phenylalkylgruppe substituiert sein können und sowohl an die 5-gliedrigen als auch an die 6-gliedrigen heteroaromatischen Ringe jeweils über zwei benachbarte Kohlenstoffatome eine n-Propylen-, n-Butylen- oder 1,3-Butadienylgruppe oder über eine Iminogruppe und ein benachbartes Kohlenstoffatom eine n-Butylen- oder 1,3-Butadienylgruppe angefügt ist und in einem so gebildeten anellierten Pyridinring eine Methingruppe durch ein Stickstoffatom und eine Vinylengruppe in 3-, 4-Stellung zu dem Stickstoffatom des gebildeten Pyridinringes durch ein Schwefelatom oder in einem so gebildeten anellierten Phenylring eine oder zwei Methingruppen durch N-Atome ersetzt sein können, wobei zusätzlich die vorstehend erwähnten ankondensierten aromatischen oder heteroaromatischen Ringe im Kohlenstoffgerüst durch ein Fluor-, Chlor- oder Bromatom,

durch eine Alkyl-, Alkoxy-, Hydroxy-, Phenyl-, Nitro-, Amino-, Alkylamino-, Dialkylamino-, Alkanoylamino-, Cyano-, Carboxy-, Alkoxycarbonyl-, Aminocarbonyl-, Alkylaminocarbonyl-, Dialkylaminocarbonyl-, Fluormethyl-, Difluormethyl-, Trifluormethyl, Alkanoyl-, Aminosulfonyl-, Alkylaminosulfonyl- oder Dialkylaminosulfonylgruppe monosubstituiert oder durch Fluor- oder Chloratome, durch Methyl-, Methoxy- oder Hydroxygruppen disubstituiert sein können sowie zwei Methylsubstituenten in 1,2-Stellung zueinander durch eine Methylen- oder Ethylenbrücke miteinander verknüpft sein können und eine gegebenenfalls in einem Imidazolring vorhandene NH-Gruppe durch eine Alkylgruppe mit 1 bis 6 Kohlenstoffatomen, durch eine Phenylalkylgruppe oder durch eine Cycloalkylgruppe substituiert sein kann, zu verstehen ist,
deren Enantiomeren und deren Salze.

**2.** Imidazo[1,2-a]pyridine der allgemeinen Formel I gemäß Anspruch 1, in der
$R_a$ eine geradkettige oder verzweigte Alkylgruppe mit 1 bis 4 Kohlenstoffatomen, eine Cyclopropyl-, Cyclobutyl-, Alkoxy-, Methoxymethyl- oder Ethoxymethylgruppe,
$R_b$ ein Wasserstoff-, Chlor- oder Bromatom, eine Alkyl-, Aminomethyl-, $R_1NHCH_2$- oder $R_1NR_2CH_2$-Gruppe, wobei
$R_1$ und $R_2$, die gleich oder verschieden sein können, Alkylgruppen mit 1 bis 4 Kohlenstoffatomen, Cyclohexyl-, Phenyl- oder Benzylgruppen oder
$R_1$ und $R_2$ zusammen eine n-Butylengruppe darstellen,
$R_c$ ein Wasserstoff-, Chlor- oder Bromatom, eine Alkyl- oder Trifluormethylgruppe,
$R_d$ ein Wasserstoffatom oder eine Alkylgruppe,
$R_e$ ein Carboxy- oder eine 1H-Tetrazol-5-yl-gruppe,
X ein Sauerstoffatom, eine gegebenenfalls durch eine Formyl-, $R_1$- oder $R_1CO$-Gruppe substituierte Iminogruppe, eine -CO-, -(HON=C)- oder -($R_3CR_4$)- Gruppe, wobei
$R_3$ ein Wasserstoffatom oder eine Alkylgruppe und
$R_4$ ein Wasserstoffatom, eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe, wobei die Heteroarylgruppe über eine Kohlenstoff-Kohlenstoff-Bindung mit der Alkoxygruppe verknüpft ist,
eine in 2-, 3- oder 4-Stellung durch eine Heteroarylgruppe substituierte Alkoxygruppe, wobei die Heteroarylgruppe über eine Kohlenstoff-Stickstoff-Bindung mit der Alkoxygruppe verknüpft ist,
eine Hydroxy-, $R_1O$-, $R_1CO$-O-, $R_1O$-CO-O-, Azido-, $R_5NR_6$-, CHO-$NR_5$-, $R_1$-CO-$NR_7$-, $R_1O$-CO-$NR_5$-, $R_5NR_6$-CO-O-, $R_1SO_2$-O-, $R_5NR_6$-CO-$NR_5$- oder $R_1SO_2$-$NR_7$-Gruppe darstellen, wobei in den vorstehend erwähnten Gruppen $R_1$ wie vorstehend erwähnt definiert ist,
$R_5$ und $R_6$, die gleich oder verschieden sein können, Wasserstoffatom oder die für $R_1$ eingangs erwähnten Bedeutungen besitzen,
$R_7$ ein Wasserstoffatom oder eine Alkylgruppe oder $R_1$ und $R_7$ zusammen eine n-Alkylengruppe mit 3 bis 5 Kohlenstoffatomen darstellen, bedeuten,
wobei, soweit nichts anderes erwähnt wurde, ein vorstehend erwähnter Alkyl- oder Alkoxyteil jeweils 1 bis 3 Kohlenstoffatome sowie ein vorstehend erwähnter Cycloalkylteil jeweils 3 bis 7 Kohlenstoffatome enthalten kann,
deren Enantiomeren und deren Salze.

**3.** Imidazo[1,2-a]pyridine der allgemeinen Formel I gemäß Anspruch 1, in der
$R_a$ eine Alkylgruppe mit 2 bis 4 Kohlenstoffatomen,
$R_b$ ein Wasserstoffatom,
$R_c$ ein Wasserstoffatom oder eine Methylgruppe,
$R_d$ ein Wasserstoffatom,
$R_e$ eine Carboxy- oder 1H-Tetrazolylgruppe und
X eine Carbonylgruppe oder eine gegebenenfalls durch eine Hydroxy-, Methoxy-, Benzyloxy-, Pyridylmethoxy-, Acetoxy-, Ethoxycarbonylmethoxy-, Cyclohexylcarbonyloxy- oder Cyclohexylaminocarbonyloxygruppe substituierte Methylengruppe bedeuten,
deren Enantiomeren und deren Salze.

**4.** Folgende Verbindungen der allgemeinen Formel I gemäß Anspruch 1:
(a) (R,S)-2-Ethyl-8-methyl-5-[α-(2'-carboxybiphenyl-4-yl)-α-hydroxy-methyl]imidazo[1,2-a]pyridin,
(b) (R,S)-2-Ethyl-8-methyl-5-[α-(2'-carboxybiphenyl-4-yl)-α-acetoxy-methyl]imidazo[1,2-a]pyridin,
(c) (R,S)-2-Ethyl-8-methyl-5-[α-(2'-carboxybiphenyl-4-yl)-α-cyclohexylaminocarbonyloxy-methyl]-imidazo[1,2-a]pyridin und

(d) (R,S)-2-n-Propyl-8-methyl-5-[α-(2'-carboxybiphenyl-4-yl)-α-hydroxy-methyl]imidazo[1,2-a]pyridin, deren Enantiomeren und deren Salze.

**5.** Physiologisch verträgliche Salze der Verbindungen nach mindestens einem der Ansprüche 1 bis 4 mit anorganischen oder organischen Säuren oder Basen.

**6.** Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

**7.** Verwendung einer Verbindung nach mindestens einem der Ansprüche 1 bis 5 zur Herstellung eines Arzneimittels mit Angiotensin-antagonistischer Wirkung.

**8.** Verfahren zur Herstellung eines Arzneimittels gemäß Anspruch 7, dadurch gekennzeichnet, daß auf nichtchemischem Wege eine Verbindung nach mindestens einem der Ansprüche 1 bis 5 in einen oder mehrere inerte Trägerstoffe und/oder Verdünnungsmittel eingearbeitet wird.

**9.** Verfahren zur Herstellung der Imidazo[1,2-a]pyridine gemäß den Ansprüchen 1 bis 5, dadurch gekennzeichnet, daß

a) zur Herstellung einer Verbindung der allgemeinen Formel I, in der X eine -(HO-$CR_3$)- Gruppe und $R_b$ ein Wasserstoff-, Fluor- oder Chloratom, eine Alkyl- oder Trifluormethylgruppe darstellt, ein Imidazo[1,2-a]pyridin der allgemeinen Formel

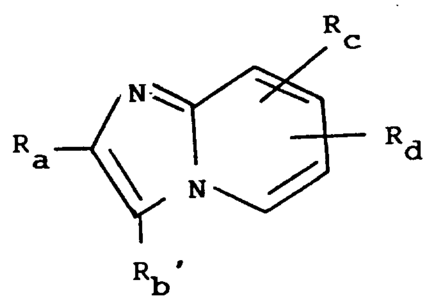

,(II)

in der

$R_a$, $R_c$ und $R_d$ wie in den Ansprüchen 1 bis 4 definiert sind und

$R_b$' ein Wasserstoff-, Fluor- oder Chloratom, eine Alkyl- oder Trifluormethylgruppe darstellt, mit einer Biphenylverbindung der allgemeinen Formel

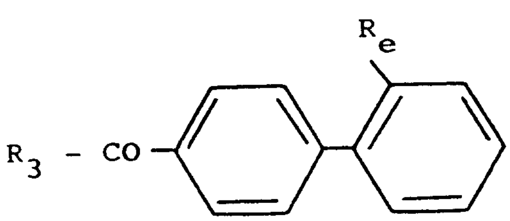

,(III)

in der

$R_e$ und $R_3$ wie in den Ansprüchen 1 bis 4 definiert sind, umgesetzt wird oder

b) zur Herstellung einer Verbindung der allgemeinen Formel I, in der $R_e$ eine Carboxygruppe darstellt, eine Verbindung der allgemeinen Formel

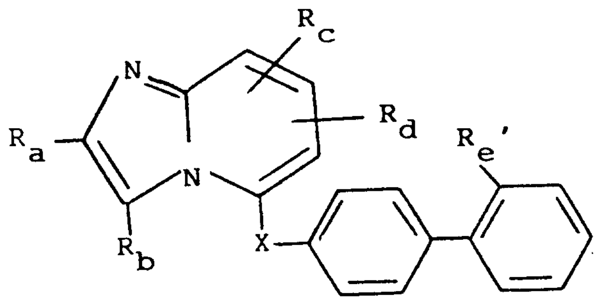

,(IV)

in der

$R_a$ bis $R_d$ und X wie in den Ansprüchen 1 bis 4 definiert sind und

$R_e'$ eine mittels Hydrolyse, Thermolyse oder Hydrogenolyse in eine Carboxygruppe überführbare Gruppe darstellt, in eine entsprechende Carboxyverbindung übergeführt wird oder

c) zur Herstellung einer Verbindung der allgemeinen Formel I, in der X eine Carbonylgruppe darstellt, eine Verbindung der allgemeinen Formel

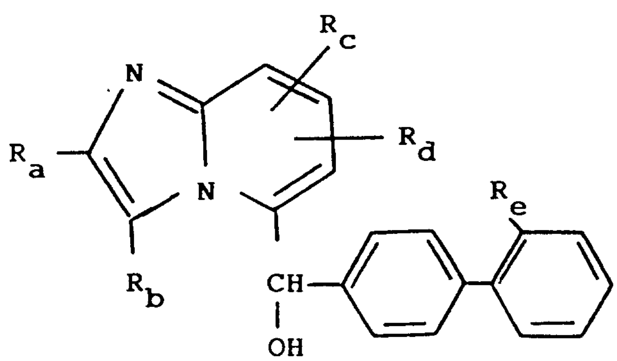

,(V)

in der

$R_a$ bis $R_e$ wie in den Ansprüchen 1 bis 4 definiert sind, oxidiert wird oder

d) zur Herstellung einer Verbindung der allgemeinen Formel I, in der X eine -($R_3$CH)- Gruppe darstellt, eine Verbindung der allgemeinen Formel

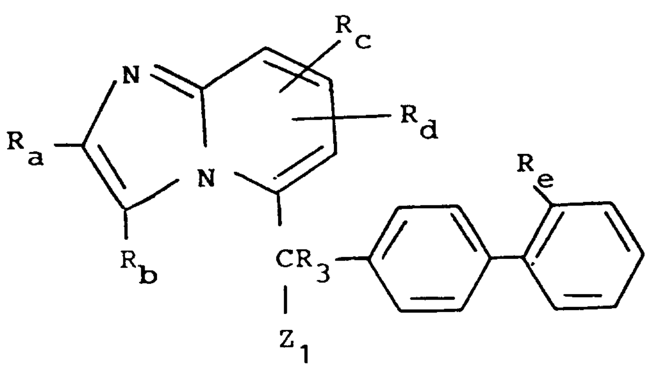

,(VI)

in der

$R_a$ bis $R_e$ und $R_3$ wie in den Ansprüchen 1 bis 4 definiert sind und

$Z_1$ eine durch ein Hydrid austauschbare Gruppe bedeutet, mit einem Organometallhydrid reduziert wird oder

e) zur Herstellung einer Verbindung der allgemeinen Formel I, in der X eine -$R_1$N- oder -($R_3$C$R_4$)-Gruppe, in denen $R_1$ mit Ausnahme der Cyclopropyl-, der Cyclobutyl- und Phenylgruppe und $R_3$ wie in den Ansprüchen 1 bis 4 definiert sind und $R_4$ eine durch eine Carboxy-, Alkoxycarbonyl-, Aminocarbonyl-, Alkylaminocarbonyl-, Dialkylaminocarbonyl- oder Heteroarylgruppe substituierte Alkoxygruppe mit 1 bis 4 Kohlenstoffatomen, wobei die Heteroarylgruppe über eine Kohlenstoff-Kohlenstoff-Bindung mit der Alkoxygruppe verknüpft ist, eine in 2-, 3- oder 4-Stellung durch eine Heteroarylgruppe substituierte Alkoxygruppe mit 2 bis 4 Kohlenstoffatomen, wobei die Heteroarylgruppe über eine Kohlenstoff-Stickstoff-Bindung mit der Alkoxygruppe verknüpft ist, eine $R_1O$- oder $R_1NR_6$-Gruppe darstellt, wobei $R_1$ und $R_3$ wie vorstehend und $R_6$ wie in den Ansprüchen 1 bis 4 erwähnt definiert sind, eine Verbindung der allgemeinen Formel

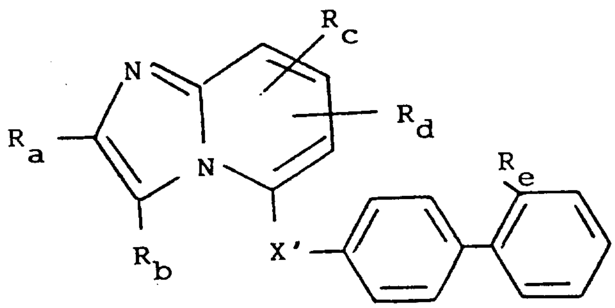

,(VII)

in der

$R_a$ bis $R_e$ wie in den Ansprüchen 1 bis 4 definiert sind und

X' eine -HN- oder -(HO-$CR_3$)- Gruppe darstellt, in welcher $R_3$ wie in den Ansprüchen 1 bis 4 definiert ist, mit einer Verbindung der Formel

$R_1' - Z_2$,   (VIII)

in der

$R_1'$ mit Ausnahme der Cyclopropyl-, Cyclobutyl- und Phenylgruppe die für $R_1$ in den Ansprüchen 1 bis 4 erwähnten Bedeutungen besitzt und zusätzlich eine durch eine Carboxy-, Alkoxycarbonyl-, Aminocarbonyl-, Alkylaminocarbonyl-, Dialkylaminocarbonyl-oder Heteroarylgruppe substituierte Alkylgruppe mit 1 bis 4 Kohlenstoffatomen, wobei die Heteroarylgruppe über eine Kohlenstoff-Kohlenstoff-Bindung mit der Alkylgruppe verknüpft ist, eine in 2-, 3- oder 4-Stellung durch eine Heteroarylgruppe substituierte Alkylgruppe mit 2 bis 4 Kohlenstoffatomen, wobei die Heteroarylgruppe über eine Kohlenstoff-Stickstoff-Bindung mit der Alkylgruppe verknüpft ist, darstellt und

$z_2$ eine nukleophile Austrittsgruppe darstellt, umgesetzt wird oder

f) zur Herstellung einer Verbindung der allgemeinen Formel I, in der X eine -($R_1$-CO-N)-, -($R_1$-CO-O-$CR_3$)-, -($R_1$O-CO-O-$CR_3$)-, -($R_1$-$SO_2$-O-$CR_3$)-, -($R_1$-CO-$NR_7$-$CR_3$)-, -($R_1$O-CO-$NR_5$-$CR_3$)- oder -($R_1SO_2$-$NR_7$-$CR_3$)- Gruppe darstellt, eine Verbindung der Formel

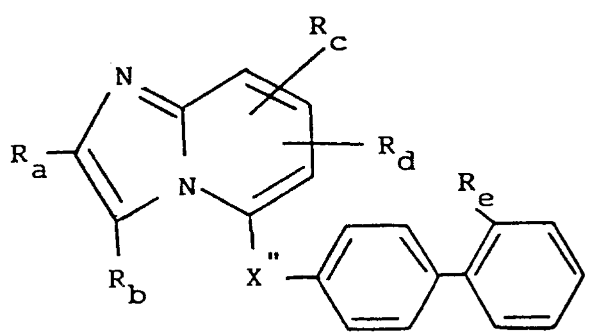

,(IX)

in der

X" eine -HN-, -(HO-$CR_3$)- oder -($R_5$NH-$CR_3$)- Gruppe darstellt, in denen $R_3$ und $R_5$ wie in den Ansprüchen 1 bis 4 erwähnt definiert sind, mit einer Verbindung der Formel

$Z_3$ - U - E,   (X)

in der
$Z_3$ eine nukleophile Austrittsgruppe,
U eine Carbonyl- oder Sulfonylgruppe und
E die für $R_1$ in den Ansprüchen 1 bis 4 erwähnten Bedeutungen besitzt oder
E zusammen mit $R_5$ eine n-Alkylengruppe mit 3 bis 5 Kohlenstoffatomen oder auch, wenn U eine Carbonylgruppe darstellt,
eine $R_1O$-Gruppe, in der $R_1$ wie in den Ansprüchen 1 bis 4 definiert ist, darstellen, oder mit deren reaktionsfähigen Derivaten wie deren Säurehalogeniden, Säureestern oder Säureanhydriden umgesetzt wird oder
g) zur Herstellung einer Verbindung der allgemeinen Formel I, in der X eine -($R_5NR_6$-CO-O-$CR_3$)- oder -($R_5NR_6$-CO-$NR_5$-$CR_3$)-Gruppe darstellt, eine Verbindung der allgemeinen Formel

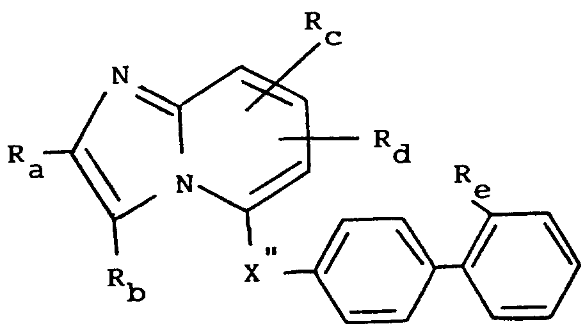

,(IX)

in der
$R_a$ bis $R_e$ und $R_3$ wie in den Ansprüchen 1 bis 4 definiert sind und X'' eine -(HO-$CR_3$)- oder -($R_5NH$-$CR_3$)- Gruppe darstellt, wobei $R_3$ und $R_5$ wie in den Ansprüchen 1 bis 4 definiert sind, mit einer gegebenenfalls im Reaktionsgemisch hergestellten Verbindung der allgemeinen Formel

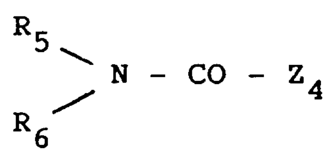

,(XI)

in der
$R_5$ und $R_6$ wie in den Ansprüchen 1 bis 4 erwähnt definiert sind und
$Z_4$ eine nukleophile Austrittsgruppe oder auch
$Z_4$ und $R_5$ zusammen eine Stickstoff-Kohlenstoff-Bindung darstellen, umgesetzt wird oder
h) zur Herstellung einer Verbindung der allgemeinen Formel I, in der $R_e$ eine 1H-Tetrazolylgruppe darstellt, ein Schutzrest von einer Verbindung der allgemeinen Formel

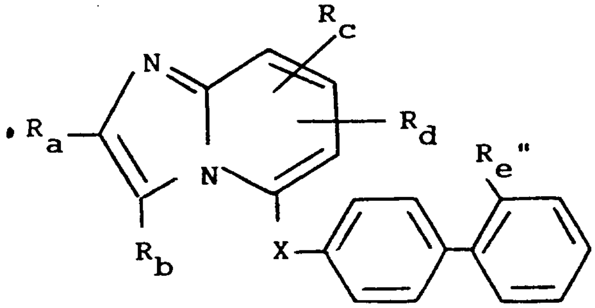

,(XII)

in der
$R_a$ bis $R_d$ und X wie in den Ansprüchen 1 bis 4 definiert sind und

$R_e$'' eine in 1- oder 2-Stellung durch einen Schutzrest geschützte 1H-Tetrazolylgruppe darstellt, abgespalten wird oder

i) zur Herstellung einer Verbindung der allgemeinen Formel I, in der X ein Sauerstoffatom oder eine $-R_5N-$ Gruppe darstellt, ein Imidazo[1,2-a]pyridin der allgemeinen Formel

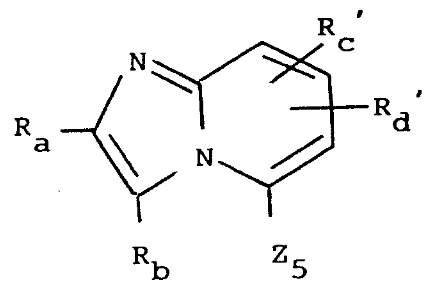

,(XIII)

in der

$R_a$ und $R_b$ wie in den Ansprüchen 1 bis 4 definiert sind,

$R_c$' ein Wasserstoffatom oder eine Alkylgruppe,

$R_d$' ein Wasserstoffatom und

$Z_5$ eine nukleophile Austrittsgruppe darstellen, mit einer Biphenylverbindung der allgemeinen Formel

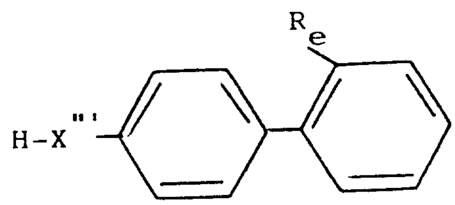

,(XIV)

in der

$R_e$ wie in den Ansprüchen 1 bis 4 definiert ist und

X''' ein Sauerstoffatom oder eine $-R_5N-$ Gruppe, in der $R_5$ wie in den Ansprüchen 1 bis 4 definiert ist, darstellt, umgesetzt wird oder

j) zur Herstellung einer Verbindung der allgemeinen Formel I, in der $R_e$ eine 1H-Tetrazolylgruppe darstellt, eine Verbindung der allgemeinen Formel

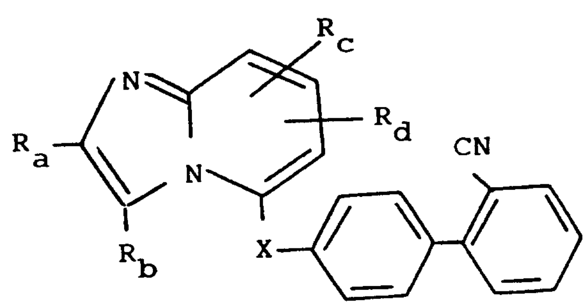

,(XV)

in der

$R_a$ bis $R_d$ und X wie in den Ansprüchen 1 bis 4 definiert sind,

mit Stickstoffwasserstoffsäure oder deren Salzen umgesetzt wird und

gewünschtenfalls anschließend eine so erhaltene Verbindung der Formel I, in der $R_b$ ein Wasserstoffatom darstellt, mittels Nitrierung in eine entsprechende Verbindung der Formel I, in der $R_b$ eine Nitrogruppe darstellt, übergeführt wird oder

eine so erhaltene Verbindung der Formel I, in der $R_b$ eine Nitrogruppe darstellt, nach Reduktion in eine entsprechende Aminoverbindung der Formel I über die entsprechenden Diazoniumsalze in eine Verbindung der Formel I, in der $R_b$ ein Wasserstoff-, Fluor-, Chlor- oder Bromatom darstellt, übergeführt wird oder

eine so erhaltene Verbindung der Formel I, in der $R_b$ einWasserstoffatom darstellt, mittels Halogenierung in eine entsprechende Verbindung der Formel I, in der $R_b$ ein Chlor- oder Bromatom darstellt, übergeführt wird oder

eine so erhaltene Verbindung der Formel I, in der $R_b$ einWasserstoffatom darstellt, mittels Umsetzung mit einem disubstituierten Formamid und Phosphoroxychlorid in eine entsprechende Verbindung der Formel I, in der $R_b$ eine Formylgruppe darstellt, übergeführt wird oder

eine so erhaltene Verbindung der Formel I, in der $R_b$ eine Formylgruppe darstellt, mittels Oxidation in eine entsprechende Verbindung der Formel I, in der $R_b$ eine Carboxygruppe darstellt, übergeführt wird oder

eine so erhaltene Verbindung der Formel I, in der $R_b$ eine Carboxygruppe darstellt, mittels Veresterung in eine entsprechende Verbindung der Formel I, in der $R_b$ eine Alkoxycarbonylgruppe darstellt, übergeführt wird oder

eine so erhaltene Verbindung der Formel I, in der $R_b$ eine Formylgruppe darstellt, mittels Umsetzung mit Hydroxylamin in ein entsprechendes Aldoxim der Formel I und durch anschließende Umsetzung des Aldoxims mit einem wasserentziehenden Mittel in eine entsprechende Verbindung der Formel I, in der $R_b$ eine Cyanogruppe darstellt, übergeführt wird oder

eine so erhaltene Verbindung der Formel I, in der $R_b$ einWasserstoffatom darstellt, mittels Umsetzung mit Ammoniak oder einem primären oder sekundären Amin und Formaldehyd in eine entsprechende Verbindung der Formel I, in der $R_b$ eine $NH_2CH_2$-, $R_1NHCH_2$- oder $R_1NR_2CH_2$-Gruppe darstellt, übergeführt wird oder

eine so erhaltene Verbindung der Formel I, in der X eine -($R_1SO_2O$-$CR_3$)-Gruppe darstellt, mittels Umsetzung mit Natriumazid in eine entsprechende Azidoverbindung der Formel I übergeführt wird oder

eine so erhaltene Verbindung der Formel I, in der $R_4$ eine Azidogruppe darstellt, mittels Reduktion in eine entsprechende Aminoverbindung übergeführt wird oder

eine so erhaltene Verbindung der Formel I, in der X eine Carbonylgruppe darstellt, mittels Umsetzung mit Hydroxylamin in die entsprechende Verbindung der Formel I, in der X eine -(HON = C)- Gruppe darstellt, übergeführt wird oder

eine so erhaltene Verbindung der Formel I, in der X eine -(HON = C)- Gruppe darstellt, mittels Reduktion in eine entsprechende Aminoverbindung der Formel I übergeführt wird oder

eine so erhaltene Verbindung der Formel I, in der X eine Carbonylgruppe darstellt, mittels Umsetzung mit 1,2-Ethandiol oder 1,3-Propandiol in eine entsprechende Verbindung der Formel I, in der X eine -($R_3CR_4$)-Gruppe darstellt, wobei $R_3$ und $R_4$ zusammen eine 1,2-Ethylendioxy- oder 1,3-n-Propylendioxygruppe darstellen, übergeführt wird und

erforderlichenfalls ein während der Umsetzungen zum Schutze von reaktiven Gruppen verwendeter Schutzrest abgespalten wird und/oder

gewünschtenfalls anschließend ein so erhaltenes Racemat in ihre Enantiomeren aufgetrennt wird oder

eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

Europäisches Patentamt

# EUROPÄISCHER RECHERCHENBERICHT

Nummer der Anmeldung

EP 93 10 2053

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 459 136 (TAKEDA)<br>* Ansprüche 1,43 *<br>----- | 1,7 | C07D471/04<br>A61K31/435<br>C07F9/6561<br>//(C07D471/04,<br>235:00,221:00) |
| | | | **RECHERCHIERTE SACHGEBIETE (Int. Cl.5)** |
| | | | C07D<br>A61K |

Der vorliegende Recherchenbericht wurde für alle Patentansprüche erstellt

| Recherchenort | Abschlußdatum der Recherche | Prüfer |
|---|---|---|
| DEN HAAG | 18 JUNI 1993 | ALFARO FAUS I. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)